(19) Europäisches Patentamt
European Patent Office
Office européen des brevets

(11) EP 0 691 963 B1

(12) **EUROPEAN PATENT SPECIFICATION**

(45) Date of publication and mention of the grant of the patent:
**15.07.1998 Bulletin 1998/29**

(21) Application number: **94912660.1**

(22) Date of filing: **28.03.1994**

(51) Int Cl.[6]: **C07D 303/38**, C07D 303/42, C07D 331/02, C07D 203/08, C07C 69/732, C07C 59/46, A61K 31/20, A61K 31/23, A61K 31/335, A61K 31/38, A61K 31/395

(86) International application number:
**PCT/IB94/00085**

(87) International publication number:
**WO 94/22848 (13.10.1994 Gazette 1994/23)**

(54) **HEPOXILIN ANALOGS USEFUL AS ANTI-INFLAMMATORY AGENTS**
HEPOXILIN ANALOGA VERWENDBAR ALS ENTZÜNDUNGSHEMMENDE MITTEL
ANALOGUES D'HEPOXILINE UTILISABLES COMME AGENTS ANTI-INFLAMMATOIRES

(84) Designated Contracting States:
**AT BE CH DE DK ES FR GB IT LI NL SE**

(30) Priority: **29.03.1993 US 38324**

(43) Date of publication of application:
**17.01.1996 Bulletin 1996/03**

(73) Proprietor: **HSC Research and Development Limited Partnership**
**Toronto, Ontario M5G 1X8 (CA)**

(72) Inventors:
- **Pace-Asciak, Cecil R.**
  **Toronto, Ontario M4T 1R5 (CA)**
- **Demin, Peter M.**
  **121019 Moscow (RU)**

(74) Representative: **Bannerman, David Gardner et al**
**Withers & Rogers**
**4 Dyer's Buildings**
**Holborn**
**London, EC1N 2JT (GB)**

(56) References cited:

- **CHEMICAL ABSTRACTS, vol. 113, no. 5, 30 July 1990, Columbus, Ohio, US; abstract no. 40257x, P.M. DEMIN ET AL. 'Synthesis and configuration assignment of hepoxilins B3' page 575 ; 'Chemical Abstracts, 12th Collective Formula Index' & BIOORG. KHIM. vol. 16, no. 1 , 1990 pages 127 - 128**
- **CHEMICAL ABSTRACTS, vol. 105, no. 7, 18 August 1986, Columbus, Ohio, US; abstract no. 58399q, C.R. PACE-ASCIAK 'Formation of hepoxilin A4, B4 and the corresponding trioxilins from 12(S)-hydroperoxy-5,8,10,14,17-eicosa pentaenoic acid' page 411, 412 ; 'Chemical Abstracts, 11th Collective Formula Index' & PROSTAGLANDINS, LEUKOTRIENES MED. vol. 22, no. 1 , 1986 pages 1 - 9**
- **TETRAHEDRON LETTERS vol. 24, no. 45 , 1983 , OXFORD GB pages 4913 - 4916 E.J. COREY ET AL. 'Total synthesis of 12-(S)-10-hydroxy-trans-11,12-epoxyeicosa-5,9 ,14-(Z)-trienoic acids, metabolites of arachidonic acid in mammalian blood platelets'**
- **SYNTHESIS no. 1 , January 1993 , STUTTGART DE pages 65 - 66 M.A. LAPITSKAYA ET AL. 'A chemoselctive synthesis of functionalized 1,4-alkadiynes (skipped diacetylenes)'**
- **TETRAHEDRON LETTERS vol. 33, no. 16 , 14 April 1992 , OXFORD GB pages 2091 - 2094 SUN LUMIN ET AL. 'Palladium mediated allylic Mitsunobu displacement: stereocontrolled synthesis of hepoxilin A3 and trioxilin A3 methyl esters'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 691 963 B1

- CHEMICAL ABSTRACTS, vol. 114, no. 5, 4 February 1991, Columbus, Ohio, US; abstract no. 42306g, P.M. DEMIN ET AL. 'Synthetic research of hepoxilins. I. Synthesis of hepoxilins B3 via the polyacetylenic approach and the configurational assignment of the carbinol center' page 701 ; 'Chemical Abstracts, 12th Collective Formula Index' & BIOORG. KHIM. vol. 16, no. 8 , 1990 pages 1125 - 1133
- TETRAHEDRON LETTERS vol. 34, no. 27 , 2 July 1993 , OXFORD GB pages 4305 - 4308 P.M. DEMIN ET AL. 'Synthesis of racemic 11,12-cyclopropyl analogs of hepoxilins A3 and B3'

## Description

It has become increasingly clear that second messengers play an important role in maintaining homeostasis in a diverse number of metabolic processes. Calcium is an important member of the group of second messengers, and regulation of calcium has become a focal point for investigating and controlling metabolic pathways and pathological conditions that can result from the aberrant regulation of these pathways. Regulation is achieved by opening or closing gated ion channels. This results in a change in the intracellular ion concentration in either of two ways: 1) changing the voltage across the plasma membrane or 2) allowing a major influx of ions, both generating an intracellular response. Calcium-regulated cell signaling pathways regulate cellular functions such as inflammation and smooth muscle contraction.

Inflammation is the body's reaction to injury. The inflammatory response involves three stages: first, an increase of blood flow to the injured area; second, an increase of capillary permeability caused by the retraction of endothelial cells lining vessel walls; and third, leucocyte migration to the site of injury. The third stage, known as chemotaxis, is a complex process that results in phagocytosis of invading agents by certain types of leucocytes such as the neutrophil. The neutrophil plays a key role in the body's response in inflammatory events such as infection. Once having arrived at the site of inflammation, the neutrophil is "activated" and releases a plethora of oxidative enzymes, known as a respiratory burst, that aid in destroying the invasive agent. An increase in intracellular calcium is thought to be involved in the initiation of the events that result in respiratory burst.

One group of compounds that have been shown to increase the influx of intracellular calcium in neutrophils is the "hepoxilins". Hepoxilins are products of an arachidonic acid pathway and have been implicated in the mediation of inflammation and smooth muscle contraction by modulation of second messenger calcium in response pathways. Hepoxilins are biologically active hydroxy epoxide derivatives of arachidonic acid formed through the 12-lipoxygenase pathway (Pace-Asciak et al., <u>J. Biol. Chem. 258:</u> 6835-6840, 1983; Pace-Asciak, <u>Biochim. Biophys. Acta 793:</u> 485-488, 1984; Pace-Asciak et al., <u>Prostaglandins 25:</u> 79-84, 1983). They are formed from 12-HPETE, an unstable hydroperoxide derivative of arachidonic acid (Pace-Asciak, <u>J. Biol. Chem. 259:</u> 8332-8337, 1984; Pace-Asciak et al., <u>Adv. Prostal. Throm. Leuk. Res. 11:</u> 133-134, 1983). Two hepoxilins have been isolated: hepoxilin $A_3$ [8(S, R)-hydroxy-11(S),12(S)-epoxy-eicosa-5Z, 9E, 14Z-trienoic acid] and hepoxilin $B_3$ [10(S, R)-hydroxy-11(S),12(S)-epoxy-eicosa-5Z, 8Z, 14Z-trienoic acid]. The term "hepoxilin" was coined in an attempt to combine aspects of structure with their first, though not necessarily their most important, demonstrated biological activity of insulin release (Pace-Asciak and Martin, <u>Prostgl. Leukotriene and Med. 16:</u> 173-180, 1984).

Hepoxilin A3 epimers are represented as:

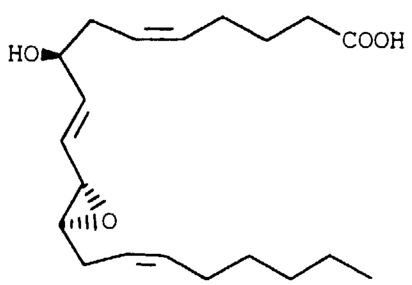

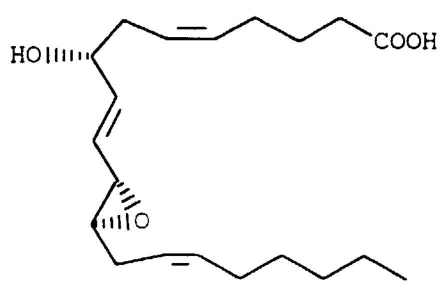

Hepoxilin B3 epimers are represented as:

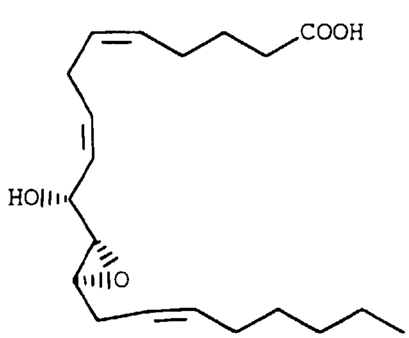

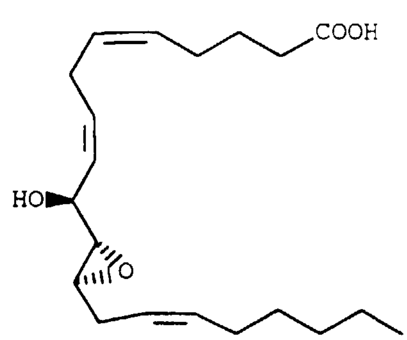

Hepoxilins are probably formed wherever 12-lipoxygenase is present because 12-HPETE is actively transformed into the hepoxilins by a variety of ferriheme proteins. Hence, ferriprotoporphyrin and such containing groups in proteins catalyze this transformation (Pace-Asciak et al., Biolog. Oxidation Systems, Eds C.C. Reddy et al., Academic Press, NY, 725-735, 1990). Hepoxilins are formed by platelets (Bryant and Bailey, Prostaglandins 17: 9-18, 1979; Jones et al., Prostaglandins 16: 583-590, 1978), lung (Pace-Asciak et al., Biochim. Biophys. Acta 712: 142-145, 1982), pancreatic islets (Pace-Asciak and Martin, ibid. 1984), brain (Pace-Asciak, ibid. 1988), aorta (Laneuville et al., Biochim. Biophys. Acta 1084: 60-68, 1991) and neutrophils (Dho et al., Biochem. J. 266: 63-68, 1990). Hepoxilin $B_3$ has been isolated from marine red algae (Moghaddam et al., J. Biol. Chem. 265: 6126-6130, 1990) and hepoxilin $A_3$ has been detected in the Aplysia brain (Piomelli et al., Proc. Natl. Acad. Sci. USA 86: 1721-1725, 1989). Hepoxilins are also formed by the rat pineal gland (Reynaud et al., J. Neurochem. 62: 126-133, 1994).

Hepoxilins have been shown to possess a variety of biological actions related to their ability to affect ion fluxes in the cell. Hepoxilins raise intracellular calcium in human neutrophils (Dho et al., Biochem. J. 266:63-68, 1990), increase the transport of calcium across membranes (Derewlany et al., Can. J. Physiol. Pharmacol. 62: 1466-1469, 1984), stimulate the release of insulin (Pace-Asciak and Martin, ibid. 1984), and regulate the volume of human platelets through an effect on potassium channels in the cell (Margalit et al. 1993 Proc. Natl. Acad. Sci. USA 90: 2589-2592, 1993). Biological actions of the hepoxilins demonstrated so far include the potentiation of aortic and tracheal vasoconstriction (Laneuville et al, Br. J. Pharmacol. 105: 297-304, 1992; 107: 808-812, 1992), potentiation of vascular permeability (Laneuville and Pace-Asciak, Prostaglandins, Leukotrienes, Lipoxins and PAF. Ed. J.M. Bailey, Plenum Press NY: 335-338, 1991), modulation of second messenger systems (Nigam et al., Biochem. Biophys. Res. Comm. 171: 944-948, 1990), regulation of cell volume (Margalit et al. Proc. Natl. Acad. Sci. USA, 90: 2589-2592, 1993 and modulation of neurotransmission (Carlen et al., Brain Res. 497: 171-176, 1989; Piomelli et al., Proc. Natl. Acad. Sci USA 86: 1721-1725, 1989; and Pace-Asciak et al., Proc. Natl. Acad. Sci. USA 87: 3037-3041, 1990).

In view of the role of the hepoxilins in regulating physiological processes, a means for regulating hepoxilin action is needed. Hepoxilin antagonists find utility in reducing inflammation, asthma, hypertension, migraine and septic shock and in modulating other processes mediated by cellular calcium levels. Hepoxilin agonists find utility in diabetes.

Figure 1 illustrates a synthetic scheme for cyclopropyl hepoxilin analogs.

Figure 2 illustrates a synthetic scheme for thiirano hepoxilin derivatives.

Figure 3 illustrates a synthetic scheme for radiolabeling hepoxilin.

Figure 4 illustrates hepoxilin analogs compete for binding on neutrophils with tritiated hepoxilin.

The present invention provides hepoxilin analogs that modulate the mobilization of intracellular calcium in human neutrophils induced by such agonists as f-Met-Leu-Phe (fMLP), platelet activation factor (PAF), leukotriene $B_4$ (LTB4), hepoxilin $A_3$ and thapsigargin. Certain of these analogs (including 11,12-cyclopropyl analogs) have been found to antagonize hepoxilin activity in experimental models. As such they may be useful in the modulation of hepoxilin-mediated (or hepoxilin agonist-mediated) processes, including inflammation associated with neutrophil activation in inflammatory disease.

Broadly speaking, the present invention is directed to hepoxilin analogs having the general structure I or II:

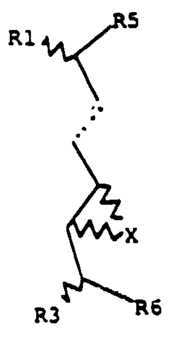
I

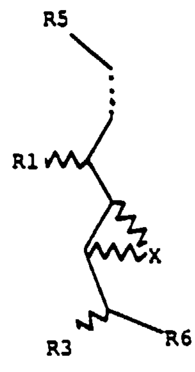
II

wherein:

X is $CH_2$, NH, or S;

R1 is OH, $CH_3$, $CH_2OH$, $N_3$ OR $CH_2N_3$;

R3 is H or $CH_3$;

R5 is Y-R2, wherein Y is a six-carbon chain optionally containing up to three double or triple bonds or a mixture of double and triple bonds up to a maximum of three;

R2 is $C_1$-$C_{10}$ alkyl-OH, $C_1$-$C_{10}$ alkyl-$N_3$ or COOR4;
R4 is H, $C_1$-$C_{10}$ alkyl, $C_5$-$C_6$ cycloalkyl, or $C_5$-$C_6$ aryl;
R6 is a seven-carbon chain optionally containing up to three double or triple bonds or a mixture of double and triple bonds up to a maximum of three; and
......... is a single, double or triple bond.

Within one embodiment, the hepoxilin analogs of the present invention are of the following structures III-IV:

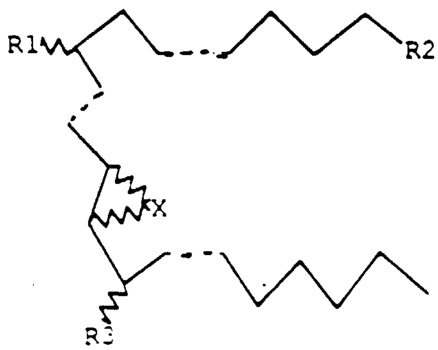

III

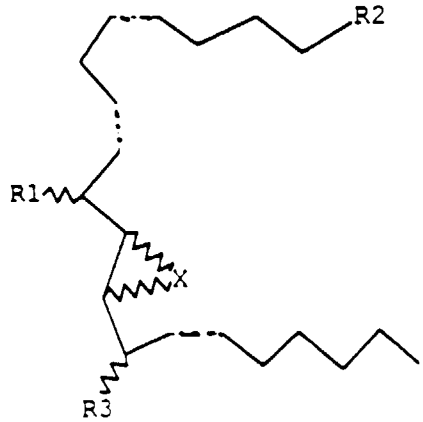

IV

wherein structures V-VI are preferred:

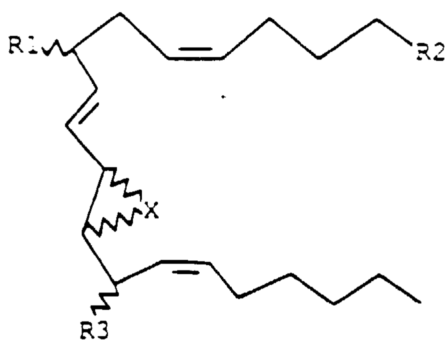

V

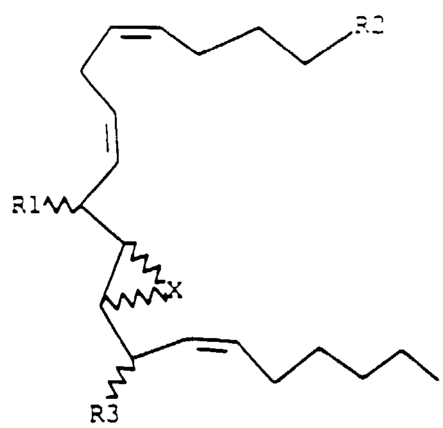

VI

Within structures III to VI, R2 is COOR4, $C_1$ - $C_{10}$ alkyl-OH or $C_1$ - $C_{10}$ alkyl-$N_3$, within structures V and VI, R2 is preferably COOR4.

Unless otherwise specified, the term "alkyl" is used herein to refer to branched and unbranched alkyl radicals. Suitable alkyl radicals include methyl, ethyl, n-propyl, isopropyl, n-butyl, tert-butyl, n-amyl, sec-amyl, n-hexyl, 2-ethylbutyl, 2,3-dimethylbutyl and the like. Lower alkyl ($C_1$-$C_6$ alkyl) radicals are preferred, such as methyl and ethyl radicals. The term "alkenyl" refers to branched and unbranched hydrocarbon chains having one or more double bonds, including mono-unsaturated and poly-unsaturated radicals. Lower alkenyl radicals having from one to six carbon atoms are preferred. The term "alkynyl" refers to branched and unbranched hydrocarbon chains having one or more triple bonds, including mono-unsaturated and poly-unsaturated radicals. Lower alkynyl radicals having from one to six carbon atoms are preferred. The term "aryl" includes monocyclic aromatic hydrocarbon radicals. Suitable aryl groups include phenyl, chlorophenyl, benzyl and the like. The term "halo" includes chloro, fluoro, bromo and iodo. The symbol is used to indicate a bond that can be single, double or triple.

Preferred epimers of hepoxilin analogs III-VI include the following:

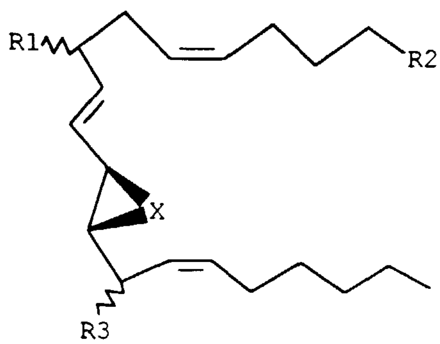

VII

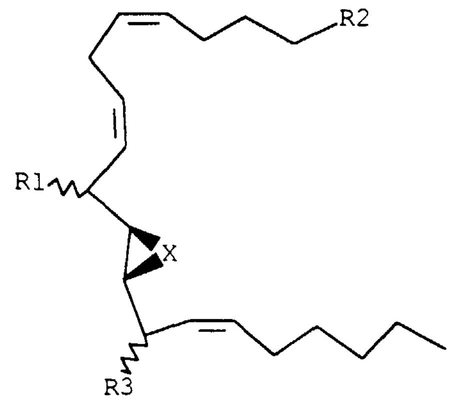

VIII

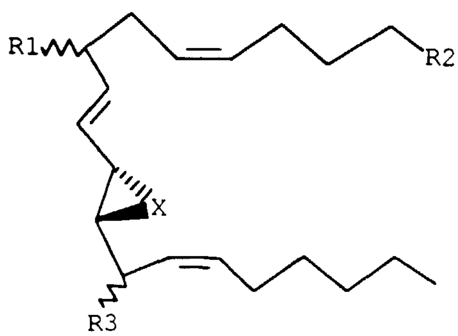

IX

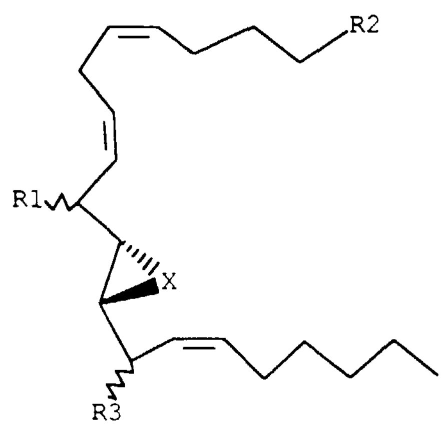

X

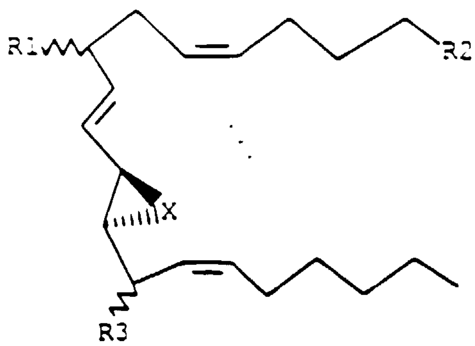

XI

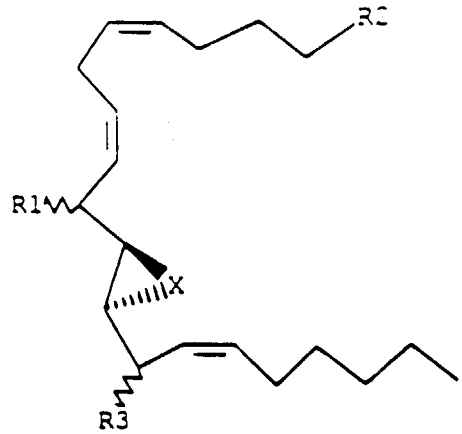

XII

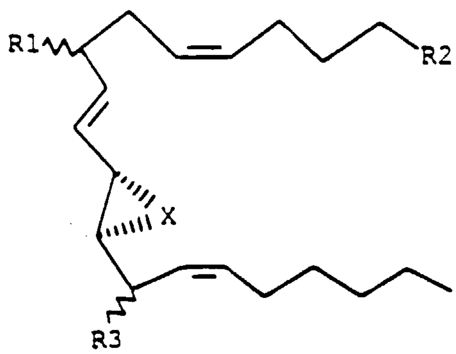

XIII

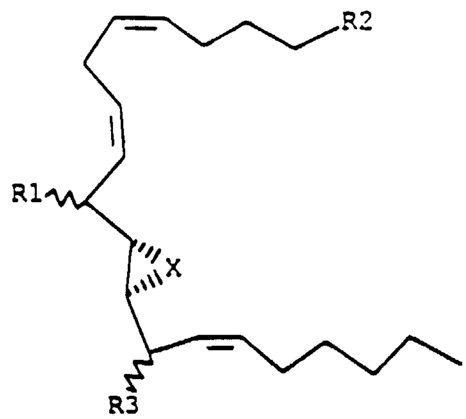

XIV

Within the compounds represented by structures III-XIV, X is NH, S or $CH_2$, R1 is OH, $CH_3$, $CH_2N_3$; R2 is COOH or $COOCH_3$; and R3 is H, $CH_3$ or $CH_2OH$. When X is $CH_2$, (forming a cyclopropane ring), X and carbon atoms 11 and 12 will form a three membered ring. Within these molecules it is particularly preferred that R1 is OH; and R3 is H.

Within another preferred group of molecules, X is $CH_2$ NH or S; R1 is OH; R2 is COOR4; R3 is H or $CH_3$; and $R_4$ is H or lower alkyl, particularly methyl. Particularly preferred compounds within this group are those in which R1= OH; X=$CH_2$; R2= COOH or $COOCH_3$; and R3=H, including those molecules designated as HA-921, HA-922, HA-923 and HA-924.

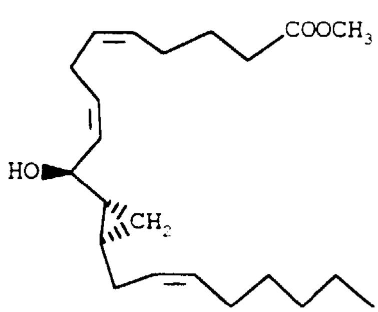

HA-921

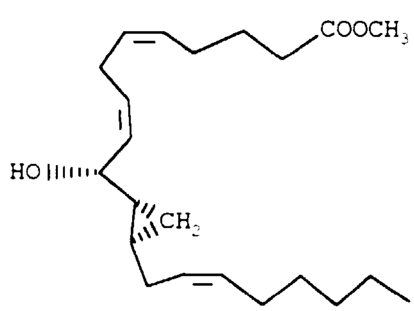

HA-922

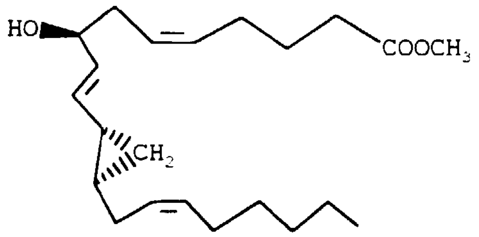

HA-923

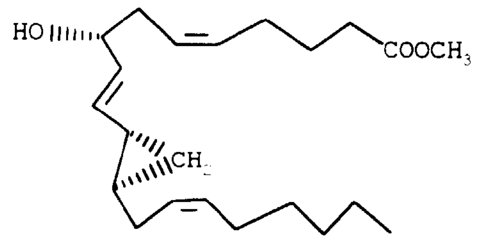

HA-924

Within another preferred group of molecules, R2 is alkyl--$N_3$, alkyl--OH, an ester of a $C_5$-$C_{10}$ alkyl radical, or an ester of an aryl radical, particularly an ester of a substituted or unsubstituted phenyl radical. Particularly preferred molecules within this group are those in which R2 is alkyl--$N_3$ or alkyl--OH, such as lower alkyl--$N_3$ or lower alkyl--OH.

According to the present invention, structural analogs of hepoxilins, including the representative hepoxilin antagonists methyl 8-hydroxy-11,12-cyclopropyl-5(Z),9(E),14(Z)-eicosatrienoate ($\Delta HxA_3$) and methyl 10-hydroxy-11,12-cyclopropyl-5(Z),8(Z),14 (Z)-eicosatrienoate ($\Delta HXB_3$), may conveniently be synthesized utilizing the acetylenic approach to hepoxilins (Demin et al., Bioorg. Khim 16:l125-l133, 1990).

A synthetic scheme for hepoxilin antagonists is set forth in Figure 1. As shown therein, 1-hydroxyundeca-2(E)-en-5-yne (1), a common intermediate in this synthetic scheme, prepared as described (Demin et al., ibid.), is modified to obtain the corresponding three-membered cyclopropane ring- containing alcohols, which are the key synthons to hepoxilin analogs. According to this scheme, alcohol (1) [1-Hydroxyundeca-2(E)-en-5-yne] is treated with $CH_2I_2$ and Zn-Cu couple in dry ether giving racemic (2S*,3S*)-2,3-cyclopropylalcohol (2), which is then oxidized to aldehyde (3) with pyrydinium dichromate. Two subsequent condensations of aldehyde (3) with Li-derivative of propargyl chloride leads to cyclopropylcarbinol (4). $^1$H-NMR spectrum at this stage shows a 7:3 ratio between two diastereomers. Propargylic chloride (4) is reacted with methyl hexynoate in the presence of equimolar amounts of CuI and NaI in DMF in the presence of $K_2CO_3$, 10 hours at 20°C, resulting in the triacetylenic analog of $\Delta HxB_3$ (5) obtained as an inseparable mixture with the same epimeric ratio. Following selective hydrogenation of the triacetylenic analog (5) on Lindlar catalyst in the presence of 2 equivalents of quinoline and subsequent separation, two $C^{10}$-epimers of more and less polar $\Delta HxB_3$ methyl esters (6a,b) are obtained in a 7:3 ratio as a colorless oil. The same ratio between more and less polar epimers is obtained for native $HxB_3$ methyl esters when similar condensations of appropriate aldehyde with Li-derivative of terminal acetylene are used (Demin et al., ibid.]. The same relative configuration for more and less polar $\Delta HxB_3$ is thus expected. This proposition is confirmed by the consideration of NMR spectra of individual $\Delta HxB_3$ methyl esters (6a, 6b). NMR spectra show the two differences between epimers: coupling constant $J_{10,11}$ is larger for the more polar epimer (d, 7.8 Hz) than for the less polar epimer (d, J 7.3 Hz), and proton at $C^{11}$ of cyclopropyl group has less chemical shift (0.68 ppm) for the more polar epimer than for the less polar epimer (0.81 ppm). These data are in agreement with those described for α,β-cyclopropylcarbinolic systems (Descotes et al., Tetrahedron 29:2931-2935, 1973). Furthermore, oxidation of both fully saturated $\Delta HxB$ ($\Delta HxB_0$) epimer and native saturated HxB ($HxB_0$) by pyrydinium dichromate into the corresponding ketones followed by treatment of the latter with sodium borohydride results, in both cases, in a mixture of initial α,β-cyclopropyl- and α,β-epoxycarbinols in a 1.7:1 ratio between less and more polar epimers. This ratio is similar to that described for reduction of α,β-epoxyketones to α,β-epoxycarbinols by $NaBH_4$ with preference of erythro- epoxycarbinol (Pierre et al., Tetrahedron Lett. (42): 4371-7374, 1972 and Nakata et al., Tetrahedron Lett. 22 (47): 4723-4726, 1981).

On the basis of the foregoing considerations, it can be concluded that the more polar $\Delta HxB_3$ epimer (6a) has a threo, syn or (10S*,11R*,12S*)-configuration, and the less polar epimer of $\Delta HxB_3$ (6b) has a relative erythro, anti or (10R*,11R*,12S*)-configuration. In the case of cyclopropyl analogs of $HxB_3$, the absolute configuration of the carbinolic center is opposite to the configuration of native $HxB_3$, while the relative configuration remains the same (Demin et al., Bioorg. Khim. 16: 1125-1133, 1990).

To obtain the two $C^8$-epimeric $\Delta HxA_3$ methyl esters (7a,b), the stereocontrolled rearrangement of allylic acetates catalyzed by Pd(II) (Grieco et al., J. Am. Chem. Soc. 102: 7587-7588, 1980) is used. Treatment of individual $\Delta HxB_3$ acetates (8a, 8b) obtaining from (6a, 6b) with 0.1 eq. of $PdCl_2(MeCN)_2$ in THF leads to the mixtures of $\Delta HxB_3$ and $\Delta HxA_3$ acetates (8a, 9a) and (8b, 9b) in a ca. 1:1 ratio in both cases. These acetates can be separated from each other by high performance liquid chromatography (HPLC) on a straight phase column (μ Porasil or the like) using 0.3% isopropanol in hexane. Subsequent hydrolysis gives two individual $C^8$-epimers of $\Delta HxA_3$ (7a, 7b). On the basis of the $S_N2'$ reaction mechanism, the more polar $\Delta HxA_3$ (obtained from anti $\Delta HxB_3$) is referred to as syn or (8R*,11S*,12S*)-epimer (7b), and the less polar $\Delta HxA_3$ as anti or (8S*,11S*,12S*) -epimer (7a), respectively. The chromatographic

properties of $\Delta HxA_3$ methyl esters (7a), (7b) are also similar to native $HxA_3$ methyl esters with known relative configuration (Demin et al., Bioorg. Khim., 16: 571-572, 1990 and Corey et al., Tetrahedron Lett.: 31(15): 2113-2116, 1990).

The above-described procedures are modified as necessary to produce other hepoxilin analogs. The necessary alterations in starting materials, reactants and conditions will be evident to those skilled in the art. Thus, the methods described above can be employed for the production of hepoxilin analogs having alternative R groups and different degrees of saturation at specific carbon atoms. Individual epimers or mixtures may be produced as desired.

Within the present invention, hepoxilin analogs may be prepared in the form of pharmaceutically acceptable salts, especially acid-addition salts, including salts of organic acids and mineral acids. Examples of such salts include organic acids such as formic acid, acetic acid, propionic acid, glycolic acid, lactic acid, pyruvic acid, oxalic acid, succinic acid, malic acid, tartaric acid, citric acid, benzoic acid, salicylic acid, tris hydroxy amino methyl (THAM) and the like. Suitable inorganic acid-addition salts include salts of hydrochloric, hydrobromic, sulfuric and phosphoric acids and the like. The acid addition salts may be obtained as the direct products of compound synthesis. In the alternative, the free base may be dissolved in a suitable solvent containing the appropriate acid, and the salt isolated by evaporating the solvent or otherwise separating the salt and solvent.

The compounds of the present invention find utility in human and veterinary medicine for modulating the action of the hepoxilins. Hepoxilin antagonists may be administered for the treatment of such conditions as inflammation, asthma, hypertension, migraine and septic shock. In general, the compounds or their salts are formulated for enteral or parenteral administration by combining them with a pharmaceutically acceptable vehicle according to conventional procedures. See, Remington's Pharmaceutical Sciences, 18th ed., Gennaro, ed., Mack Publishing Co, Easton, PA, 1990, incorporated herein by reference. The active ingredient will be combined with one or more diluents, fillers, emulsifiers, excipients, etc., and may be provided in such forms as liquids, powders, emulsions, tablets, capsules and the like. Such compositions may further include one or more auxiliary substances, including wetting agents, preservatives, stabilizers, buffers, lubricants, colorings, etc.

The following non-limiting examples are provided to illustrate certain embodiments of the invention.

Example I

Instruments and reagents

Referring to Figure 1, vacuum distillation of compound (2) was done using a Kugelrohr apparatus (Aldrich, Milwaukee, WI) at the stated oven temperature. Thin layer chromatography was performed on aluminum sheets coated with silica gel 60 $F_{254}$, layer thickness 0.2 mm (Merck, Darmstadt, Germany). Preparative HPLC was performed using a μ Porasil $SiO_2$ column, 7.8 x 300 mm (Waters, Milford, MA), using as eluent 0.8% i-PrOH in hexane for compounds (6a,b) and (7a,b), 0.3% i-PrOH in hexane for (8a,b) and (9a,b). GC analyses were carried out on a Hewlett-Packard 5700A gas chromatograph (Hewlett-Packard, Palo Alto, CA) using a glass capillary column, (SPB-1, Supelco, Bellefonte, PA) 60 m x 0.3 mm. Electron impact mass spectra were obtained on a Hewlett-Packard GC MS using a fused silica methyl silicone capillary column (HP-1, 12 m x 0.2 mm). Compounds (4), (5), (6a,b) and (7a,b) were injected onto the GC column as their tBDMSi derivatives prepared with a t-butyldimethylsilylimidazole-DMF kit (Supelco). $^1H$-NMR spectra were obtained on Bruker-AM500 (500 MHz) spectrometer (Bruker, Karlsruhe, Germany) in $CDCl_3$ with $Me_4Si$ as an internal standard ($\delta$=0).

Pyridinium dichromate, Lindlar catalyst (Aldrich) and n-BuLi solution in hexane (Fluka, Buchs, Switzerland) were used as received. 1-Hydroxyundec-2(E)-en-5-yne (1) and 5-hexynoic acid were synthesized as described (Demin et al., Bioorg. Khim. 16: 1125-1133, 1990).

(2S*,3S*)-1-Hydroxyundec-2,3-Cyclopropyl-5-Yne (2)

A suspension of Zn-Cu couple (2.9 g) in dry ether (10 mL) in the presence of $I_2$ (0.1 g) was refluxed for 5 min until the red-brown color disappeared. Diiodomethane (2.95 g) was added, and the resulting mixture was stirred 1 h at 35°C. l-Hydroxyundec-2(E)-en-1-yne (1; numbers refer to the synthetic scheme shown in the Figure) (0.33 g) in 5 mL of ether was added, and the reaction mixture was refluxed for 30 min with stirring. Saturated aqueous $NH_4Cl$ was added, the mixture was extracted with ether, and the ether phase was dried over $Na_2SO_4$. After evaporation of the solvent, the residue was distilled in vacuo, yielding 279 mg (78%) of cyclopropyl alcohol (2), b.p. 100-110°C ($10^{-2}$ mm Hg). $^1H$-NMR (500 MHz, $\delta$, ppm): 0.43, 0.53 (dt, 1H, J 4.9 and 8.5 Hz, cyclopropyl-H), 0.84, 1.05 (m, 2H, $H^2 + H^3$), 0.90 (t, 3H, J 7.0 Hz, $H^{11}$), 1.33 (m, 4H, $H^9 + H^{10}$), 1.48 (quintet, 2H, J 7.22 Hz, $H^8$), 2.14 (tt, 2H, J 2.4 and 7.2 Hz, $H^7$), 2.19 (dt, 0.5 H, J 2.4 and 6.0 Hz, $H^4$), 2.23 (dt, 0.5 H, J 2.4 and 6.0 Hz, $H^4$), 2.32 (dt, 0.5 H, J 2.4 and 5.5 Hz, $H^4$), 2.35 (dt, 0.5 H, J 2.4 and 5.5 Hz, $H^4$), 3.46 (m, 2H, $H^1$). Mass spectrum (m/z, % of related intensity): 162 ($[M - H_2O]^+$ 0.4), 109 ($[M - C_4H_9]^+$, 25), 95 ($[M - C_5H_{11}]^+$, 34), 91 ($[M - C_4H_9 - H_2O]^+$, 67), 79 (100), 77 ($[M - C_5H_{11} - H_2O]^+$, 52).

(2R*,3S*)-2,3-Cyclopropylundec-5-Yn-1-Al (3)

To a suspension of $Py_2H_2Cr_2O_7$ (85 mg) and molecular sieves 3Å (Aldrich) (120 mg) in 3 mL of dry $CH_2Cl_2$, 10 µl of AcOH and cyclopropyl alcohol (2) in 0.5 mL of $CH_2Cl_2$ was added. After 30 min, the reaction was quenched by addition of 20 µl of isopropanol, and the mixture was filtered through silica gel and eluted with hexane. Evaporation of the solvent gave 18.2 mg (92%) of aldehyde (3). $^1$H-NMR (500 MHz, δ, ppm): 0.89 (t, 3H, J 6.9 Hz, $H^{11}$), 1.15, 1.30 (m, 2H, cyclopropyl-H), 1.33 (m, 4H, $H^9 + H^{10}$), 1.48 (quintet, 2H, J 7.1 Hz, $H^8$), 1.65, 1.88 (m, 2H, $H^2 + H^3$), 2.12 (tt, 2H, J 2.2 and 7.1 Hz, $H^7$),.2.45 (m, 2H, $H^4$), 9.13 (d, 1H, J 5.3 Hz, $H^1$). Mass-spectrum (m/z, % of related intensity): 177 ($[M - 1]^+$, 0.4), 149 ($[M - CHO]^+$, 5.0), 121 ($[M - C_4H_9]^+$, 10), 107 ($[M - C_5H_{11}]^+$, 25), 93 ($[M - C_4H_9 - CHO + H]^+$, 29), 79 ($[M - C_5H_{11} - CHO + H]^+$, 47), 69 (100).

(4S*, 5R*, 6S*)- and (4R*, 5R*, 6S*)-1-Chloro-4-Hydroxy5.6-Cyclopropyltetradeca-2,8-Diynes (4)

A solution of propargyl chloride (1.6 g) in dry ether (100 mL) was cooled to -78°C, and n-BuLi (1.38 g, 21.6 mL of 1.5 M in hexane) was added over a period of 15 min. The mixture was stirred for 30 min at -78°C, and aldehyde (3) (0.77 g) in 20 ml of ether was added. After 15 min, 20 ml of $H_2O$ was added to quench the reaction, and the mixture was warmed to 20°C. The ether layer was separated, the residue was extracted with ether, the combined extracts were dried over $Na_2SO_4$, and the solvent was evaporated to dryness resulting in an orange oil which was absorbed on a silica gel column. Elution with 10% EtOAc in hexane and solvent removal gave an epimer mixture of racemic α,β-cyclopropyl alcohols (4) as a colorless oil (0.91 g, 84%, epimer ratio 7:3 by $^1$H-NMR analysis); $^1$H-NMR (500 MHz, δ, ppm); 0.57-0.69 (m, 2H, cyclopropyl-H), 0.90 (t, 3H, J 7.1 Hz, $H^{14}$), 1.03, 1.09 (m, 1 H, $H^5$), 1.19 (m, 1H, $H^6$), 1.32 (m, 4H, $H^{12} + H^{13}$), 1.48 (quintet, 2H, J 7.2 Hz, $H^{11}$), 1.89 (d, 1H, J 6.4 Hz, OH), 2.13 (tt, 2H, J 7.2 and 2.4 Hz, $H^{10}$), 2.22-2.40 (m, 2H, $H^7$), 4.15 (t, 2H, J 2.3 Hz, $H^1$), 4.28 (m, 0.3 H, $H^4$), 4.35 (m, 0.7 H, $H^4$). Mass spectrum of the tBDMSi derivative (m/z, % of related intensity): 365 ($[M - 1]^+$, 0.15), 331 ($[M - Cl]^+$, 1.9), 309 ($[M - t\text{-}Bu]^+$, 5.0), 275 ($[M - t\text{-}Bu - Cl + H]^+$, 6.8), 199 ($[M - Cl - tBDMSi]^+$, 24), 129 ($[M - C_5H_{11} - Cl - t\text{-}BuMe_2SiOH + H]^+$, 92), 128 (100).

Methyl (10S*, 11R*, 12S*)- and (10R*, 11R*, 12S*)-10-Hydroxy-11,12-Cyclopropyleicosa-5,8,14-Triynoates (5)

To a suspension of anhydrous CuI (490 mg), NaI (774 mg) and $K_2CO_3$ (712 mg) in DMF (50 mL), the solutions of methyl 5-hexynoate (390 mg in 5 mL of DMF) and propargylic chloride (4) (650 mg in 5 mL of DMF) were successively added. The mixture was stirred overnight at 20°C, then saturated aqueous $NH_4Cl$ solution (200 mL) was added. After extraction with ether, the organic layer was dried by $Na_2SO_4$, and the solvent was evaporated to dryness. Subsequent filtration of the resulting oil through silica gel (eluent 15% EtOAc in hexane) afforded triyne (5) as a yellow oil (740 mg, 84%, epimer ratio 7:3 by $^1$H-NMR analysis). $^1$H-NMR (500 mHz, δ, ppm): 0.57, 0.64 (m, 2H, cyclopropyl-H), 0.90 (t, 3H, J 7.0 Hz, $H^{20}$), 1.00-1.22 (m, 2H, $H^{11} + H^{12}$), 1.33 (m, 4H, $H^{18} + H^{19}$), 1.48 (quintet, 2H, J 7.1 Hz, $H^{17}$), 1.82 (quintet, 2H, J 7.2 Hz, $H^3$), 2.13-2.25 (m, 6H, $H^4 + H^{13} + H^{16}$), 2.44 (t, 2H, J 7.44 Hz, $H^2$), 3.15 (quintet, 0.8H, J 2.3 Hz, $H^7$), 3.30 (br.t., 0.2H, J 2.3 Hz, $H^7$), 3.68 (s, 3H, COOMe), 4.26 (dt, 0.3H, J 6.4 and 1.3 Hz, $H^{10}$), 4.32 (dt, 0.7H, J 6.1 and 1.9 Hz, $H^{10}$). Mass spectrum of the tBDMS derivative (m/z, % of related intensity): 455 ($[M - 1]^+$, 0.15), 425 ($[M - OMe]^+$, 0.19), 399 ($[M - t\text{-}Bu]^+$, 37), 293 ($[C^{10}\text{-}C^{20}]^+$, 3.7), 317 ($[C^8\text{-}C^{20}]^+$, 0.90), 307 ($[C^1\text{-}C^{10}]^+$, 0.80), 221 (64), 189 ($[(C^1\text{-}C^{11}) - t\text{-}BuMe_2SiOH + H]^+$, 17), 75 (100).

Methyl (10S*, 11R*, 12S*)- and (10R*, 11R*, 12S*)-10-Hydroxy,11,12-Cyclopropyl-5(Z),8(Z),14(Z) -Eicosatrienoates (6a,b)

A solution of triyne (5) (120 mg) in dry benzene (2 mL) was added to a mixture of hydrogen-presaturated Lindlar catalyst (100 mg) and quinoline (1000 µl) in 10 ml of benzene, and the resulting mixture was stirred until hydrogen consumption was completed (60 min, 10 mL of $H_2$ was absorbed). The poisoned catalyst was filtered off, and the filtrate was added to a new portion of hydrogen-presaturated Lindlar catalyst (100 mg) and quinoline (200 µl). After hydrogen consumption was stopped the procedure was repeated twice. During this procedure, the reaction mixture was analyzed by GC-MS as the tBDMSi derivative monitoring the prominent ion m/z 405 $[M - t\text{-}Bu]^+$. After the reaction was completed (total hydrogen absorption 79.4 mL, 340% of theoretical amount), GC MS analysis of the reaction mixture showed 92% conversion into the desirable trienes (6a, b). The catalyst was filtered off, the filtrate was poured onto an aluminum oxide column (pH 6.9-7.1). Quinoline was removed with 5% EtOAc in hexane. The column was then eluted with EtOAc, giving a yellow oil (115 mg) containing 95% trienes (6a,b) (evaluated by GLC analysis of tBDMSi derivative). Individual epimers (6a), (6b) were separated using HPLC (µPorasil 7.8 x 300 mm, Waters), eluent 1.0% i-PrOH in hexane, UV-detection at 210 nm. More polar epimer (6a): a colorless oil, 55 mg yield (46%). Rf 0.39 ($C_6H_6$-$Et_2O$, 85:15, 3 developments), $^1$H-NMR-spectrum (500 MHz, δ, ppm): 0.39 (dt, 1H, J 4.8 and 8.1 Hz, cyclopropyl-H), 0.53 (dt, IH, J 4.8 and 8.5 Hz, cyclopropyl-H), 0.68 (m, 1H, $H^{11}$), 0.83 (m, 1H, $H^{12}$), 0.87 (t, 3H, J 6.8 Hz, $H^{20}$), 1.26-1.36 (m, 6H, $H^{17}$

+ $H^{18}$ + $H^{19}$), 1.63 (br.s., 1H, OH), 1.70 (quintet, 2H, J 7.5 Hz, $H^3$), 1.90-2.12 (m, 6H, $H^4$ + $H^{13}$ + $H^{16}$), 2.32 (t, 2H, J 7.5 Hz, $H^2$), 2.75 (m, 1H, $H^7$), 2.86 (m, 1H, $H^7$), 3.67 (s, 3H, COOMe) 3.95 (ddd, 1H, J 1.0, 7.8 and 7.8 Hz, $H^{10}$), 5,36-5.50 (m, 6H, olefinic H). Mass spectrum, tBDMSi derivative (m/z, % of related intensity): 462 ($[M]^+$, 0.06), 431 ($[M - OMe]^+$, 0.80), 405 ($[M - t\text{-}Bu]^+$, 37), 324 ($[C^1\text{-}C^{11}]^+$, 20), 211 (12), 169 (12), 105 (21), 75 (100). Less polar epimer (6b): a colorless oil, 30 mg yield (25%). $R_f$ 0.51 ($C_6H_6$-$Et_2O$, 85:15, 3 developments), $^1$H-NMR-spectrum (500 MHz, δ, ppm): 0.33 (dt, 1H, J 5.1 and 8.4 Hz, cyclopropyl-H), 0.43 (dt, 1H, J 5.1 and 8.4 Hz, cyclopropyl-H), 0.81 (m, 2H, $H^{11}$ + $H^{12}$), 0.89 (t, 3H, J 6.7 Hz, $H^{20}$), 1.25-1.36 (m, 6H, $H^{17}$ + $H^{18}$ + $H^{19}$), 1.58 (d, 1H, J 3.2 Hz, OH), 1.70 (quintet, 2H, J 7.4 Hz, $H^3$), 1.96-2.10 (m, 6H, $H^4$ + $H^{13}$ + $H^{16}$), 2.31 (t, 2H, J 7.4 Hz, $H^2$), 2.74, 2.84 (m, 2H, $H^7$), 3.67 (s, 3H, COOMe), 3.97 (ddd, 1H, J 3.0, 7.3, and 7.3 Hz, $H^{10}$), 5.36-5.48 (m, 6H, olefinic H). Mass spectrum, tBDMSi derivative (m/z, % of related intensity): 462 (0.04), 431 (0.35), 405 (20), 334 (4.7), 324 (2.8), 215 (4.2), 211 (3.0), 169 (6.0), 105 (26), 75 (100).

Methyl (8S*, 11S*, 12S*)- and (8R*, 11S*, 12S*)-8-Hydroxy-11,12-Cyclopropyl-5(Z),9(E),14(Z) -Eicosatrienoates (7a,b)

A mixture of triene (6a), (6b) (10 mg of each epimer), acetic anhydride (150 µl), and pyridine (450 µl) was maintained at 20°C overnight. The mixture was evaporated to dryness, and the residue was purified by HPLC using 0.8% i-PrOH in hexane as eluent. The resulting allylic acetates (8a), (8b) were treated separately with a catalytic amount of freshly prepared bis(acetonitrile)palladium(II) chloride (0.6 mg, 0.1 equiv) in dry THF (10 mL) during 3 h at 20°C. THF was evaporated, and the residue was passed through an $Al_2O_3$ column (pH 6.9-7.1) using EtOAc as eluent. The resulting yellow oil contained a mixture of acetates $\Delta HxA_3$ (7a), (7b) and $\Delta HxB_3$ (6a), (6b) (ratio 1.2:1 from more polar $\Delta HxB_3$ (HA-922) epimer (6a) and 0.9:1 from less polar $\Delta HxB_3$ (HA-921) epimer (6b) by HPLC analysis). Acetates were separated by HPLC using 0.3% i-PrOH in hexane as eluent, yield of (9a) was 5.8 mg, 58% (from more polar $\Delta HxB_3$ (6a)), and (9b) was 3.8 mg, 38% (from less polar $\Delta HxB_3$ (6b)); $\Delta HxA_3$ acetates (9a), (9b) were non-separable from each other by HPLC or by TLC. $^1$H-NMR spectrum (any epimer, 500 MHz, δ, ppm) : 0.57 (m, 2H, cyclopropyl-H), 0.80, 1.15 (m, 2H, $H^{11}$ and $H^{12}$), 0.88 (t, 3H, J 6.91 Hz, $H^{20}$), 1.28-1.32 (m, 6H, $H^{17}$ + $H^{18}$ + $H^{19}$),1.69 (quintet, 2H, J 7.4 Hz, $H^3$), 1.99-2.07 (m, 6H, $H^2$ + $H^4$ + $H^{16}$), 2.01 (s, 3H, OAc), 2.32 (m, 4H, $H^7$ + $H^{13}$), 2.37 (dt, 1H, J 7.0 and 7.2 Hz, $H^8$), 3.67 (s, 3H, COOMe), 5.19 (dt, 1H, J 6.7 and 6.8 Hz, $H^6$), 5.29 (dd, 1H, J 8.7 and 15.5 Hz, $H^{10}$), 5.38 (m, 3H, $H^5$ + $H^{14}$ + $H^{15}$), 5.44 (dd, 1H, J 7.2 and 15.5 Hz, $H^9$). Hydrolysis of the resulting pure acetates (9a), (9b) (10% NaOH/$H_2O$ in THF, 5 h, 20°C) followed by esterification by diazomethane provided diastereochemically pure $\Delta HxA_3$ methyl esters (7a), (7b). More polar epimer of $\Delta HxA_3$ (HA-924) (7b) (from less polar $\Delta HxB_3$ (6b)): 5.1 mg yield (51%). Rf 0.46 (C6H6-Et2O, 85:15, 3 developments), mass spectrum of the tBDMSi derivative (m/z, % of related intensity): 431 ($[M - OMe]^+$, 0.18), 405 ($[M - t\text{-}Bu]^+$, 4.7), 351 ($[C^1\text{-}C^{12}]^+$, 0.18), 321 ($[C^8\text{-}C^{20}]^+$, 100), 197 (62), 189 ([($C^8$-$C^{20}$) - t-BuMe$_2$SiOH, 17), 171 (27), 75 (79), 73 (83). Less polar epimer of $\Delta HxA_3$ (HA-923) (7a) (from more polar $\Delta HxB_3$ (6a)): yield is 3.3 mg (33%). Rf 0.50 ($C_6H_6$-$Et_2O$, 85:15, 3 developments), mass spectrum of the tBDMSi derivative (m/z, % of related intensity): 431 (0.06), 405 (1.0), 351 (0.04), 321 (28), 197 (24), 189 (7.3), 171 (12), 75 (56), 73 (100).

Example II

We used the same acetylenic approach as in Example I to form Hepoxilins B with an unnatural epoxy group, i.e. 11R, 12R. These compounds were inverted under Mitsunobu conditions into a mixture of unnatural $HxB_3$ benzoates as well as the products of allylic transposition, unnatural $HxA_3$ benzoates. Exchange of O for S in the unnatural $HxA_3$ benzoates resulted in inversion of both C11 and C12 centers to afford the thiirano analog of each of the 8R and 8S epimers. The following detailed description provides an example for the synthesis of the thiirano hepoxilin $A_3$ analogs.

Thin layer chromatography (TLC) was performed on aluminum sheets coated with silica gel 60 F 254, layer thickness 0.2 mm (Merck). HPLC was performed using a µ Porasil $SiO_2$ column, 3.9 x 300 mm for analysis and 7.8 x 300 mm for preparative separation (Waters), using as eluent 0.7% i-PrOH in hexane for compounds (10a,b; numbers refer to Figure 2) and (13a,b), 0.3% i-PrOH in hexane for (11a,b) and (12a,b). $^1$H-NMR spectra were obtained on a Bruker AM 500 (500 MHz) spectrometer (Bruker) in $CdCl_3$ with $Me_4Si$ as an internal standard (δ=0). EI mass spectra were obtained on a Hewlett-Packard GC MS (Hewlett-Packard) using a fused silica methyl silicone capillary column (HP-1, 12 m x 0.2 mm). Compounds (13a,b) were injected onto the GC column as their t-BDMSi derivatives prepared with a t-butyldimethylsilylimidazole-DMF kit (Supelco).

Diethylazodicarboxylate (DEAD), triphenylphosphine, benzoic acid and potassium thiocyanate (Aldrich) were used as prepared by manufacturer. Methyl 10(R,S)-hydroxy-11(R),12(R)-epoxyeicosa-5Z,8Z,14Z-trienoates (10a,b) (unnatural epimeric $HxB_3$) were synthesized in an analogous manner to the natural epimers (Vasiljeva et al., Tetrahedron 49: 4099-4106, 1993).

Methyl 8(R,S)-benzyloxy-11(R),12(R)-epoxyeicosa-5Z,9E,14Z-trienoates (11a,b) and methyl 10(R,S)-benzyloxy-11 (R),12(R)-epoxyeicosa-5Z,8Z,14Z-trienoates (12a,b) (reference compound)

To the solution of 35 mg (100 μM) of unnatural $HxB_3$ methyl esters (10a), (10b) as individual epimers in 5 ml of dry THF, PhCOOH, DEAD and then $PPh_3$ (150 μM each) were added successively at 20°C. After 10 minutes, the reaction mixture was analyzed by TLC. Analysis showed the complete conversion into the mixture of $HxA_3$ and $HxB_3$ benzoates (11a,b), (12a,b) ($R_f$ 0.52 and 0.56, EtOAc-Hx, 3:7). The reaction was quenched by addition of 100 μl of MeOH, and the solvent was taken into dryness. The residue was passed through silica gel to remove the reagents, and the mixture of $HxA_3$ and $HxB_3$ benzoates was eluted with EtOAc-Hx, 1:9 and separated on straight-phase HPLC, UV-detection at λ 228 nm. The combined yield of $HxA_3$ and $HxB_3$ benzoates after purification was 95%. UV-spectrum of compounds (11a,b), (12a,b): $\lambda_{max}$ 228 nm. $^1$H-NMR spectrum, methyl $HxA_3$ benzoate (11a,b) (any epimer, 500 MHz, δ, ppm): 0.88 (t, 3H, J 7.1 Hz, $H^{20}$), 1.23-1.36 (m, 6H, $H^{17}$ + $H^{18}$ + $H^{19}$), 1.68 (quintet, 2H, J 7.3 Hz, H3), 2.01 (dt, 2H, J 7.3 and 6.7 Hz, $H^4$), 2.10 (dt, 2H, J 7.2 and 6.7 Hz, $H^{16}$), 2.30 (t, 2H, J 7.5 Hz, $H^2$), 2.38-2.55 (m, 4H, $H^7$ + $H^{13}$), 2.84-2.87 (m, 1H, $H^{12}$), 3.14 (dd, 1H, J 7.5 and 2.1 Hz, $H^{11}$), 3.66 (s, 3H, $COOCH_3$), 5.38 (br. ddt, 1H, J 1.5, 7.5, and 18.2 Hz, $H^{10}$), 5.44-5.58 (m, 5H, $H^5$ + $H^6$ + $H^8$ + $H^{14}$ + $H^{15}$), 5.97 (dd, 1H, J 6.4 and 15.4 Hz, $H^9$), 7.44, 7.56 and 8.04 (m, 5H, $C_6H_5$). $^1$H-NMR spectrum, methyl (10R)-*erythro*-$HxB_3$ benzoate (12b) (500 MHz, δ, ppm): 0.88 (t, 3H, J 6.9 Hz, $H^{20}$), 1.23-1.36 (m, 6H, $H^{17}$ + $H^{18}$ + $H^{19}$), 1.7 (quintet, 2H, J 7.5 Hz, $H^3$), 2.01 (dt, 2H, J 7.5 and 6.2 Hz, $H^4$), 2.12 (dt, 2H, J 7.3 and 6.2 Hz, $H^{16}$), 2.28 and 2.42 (m, 2H, $H^{13}$ + $H^{13'}$), 2.32 (t, 2H, J 7.5 Hz, $H^2$), 2.96 (m, 2H, $H^7$ + $H^{7'}$), 3.00 (dt, 1H, J 5.4 and 2.1 Hz, $H^{12}$), 3.04 (dd, 1H, J 2.1 and 3.6 Hz, $H^{11}$), 3.66 (s, 3H, $COOCH_3$), 5.34-5.55 (m, 5H, olefinic H), 5.68 (ddt, 1H, J 0.8, 10.9 and 7.5 Hz, $H^8$), 5.92 (ddd, 1H, J 0.8, 3.6 and 9.1 Hz, $H_{10}$), 7.43, 7.56 and 8.84 (m, 5H, $C_6H_5$). $^1$H-NMR spectrum, methyl (10S)-*threo*-$HxB_3$ benzoate (12a) (500MHz, δ, ppm): 2.97 (m, 3H, $H^7$ + $H^{12}$), 3.08 (dd, 1H, J 2.2 and 5.7 Hz, $H^{11}$), 5.64-5.69 (m, 2H, $H^8$ + $H^{10}$).

The ratio between $HxA_3$ and $HxB_3$ benzoates was established by the removal of benzoate groups (5 ml of 5% MeONa in MeOH, 2 hours, 20°C) followed by HPLC analysis (column μ Porasil 3.9 x 300 mm, eluent 0.7% i-PrOH in hexane, detection at λ 210 nm). Methyl (10S,11R,12R)-*threo*-$HxB_3$ have 59% of the inverted methyl (10R,11R,12R)-erythro-$HxB_3$ and 41% of the 6:1 mixture of methyl (8S,11R,12R)-*syn*- and (8S,11R,12R)-*anti*-HxA3 whereas methyl (10R,11R,12R)-*erythro*-$HxB_3$ led to 90% of the methyl (10S,11R,12R)-*threo*-$HxB_3$ and 10% of the 1:6 mixture of methyl (8S,11R,12R)-*syn*- and (8R,11R,12R)-*anti*-$HxA_3$.

Methyl 8(R,S)-hydroxy-11(S),12(S)-thiiranoeicosa-5Z,9E,14Z-trienoates (13a,b) (thio $HxA_3$ analogs).

A mixture of methyl 8(R,S)-benzyloxy-11,12-epoxyeicosa-5Z,9E,14Z-trienoates (11a,b) (50 mg, 0.11 mmol) and anhydrous KSCN (600 mg, 6.1 mmol) in dry methanol was heated to reflux for 7 hours. The 15% solution of MeONa in MeOH was then added and the reaction mixture was stirred overnight at 20°C. Methanol was taken to dryness, and the mixture was dissolved in $H_2O$/NaCl saturated solution and extracted with ether. The combined organic extracts were washed with water and dried. Solvent removal gave an oily residue which was purified on a $SiO_2$ column, eluent EtOAc-Hx, 1:2. Final purification and epimer separation was performed on HPLC, UV-detection at λ 210 nm, giving thio analogs of $HxA_3$ (13a), (13b) as individual epimers, yield 27 mg (68%). $^1$H-NMR spectrum (any epimer, 500 MHz, δ, ppm): 0.89 (t, 3H, J 6.84 Hz, $H^{20}$), 1.24-1.38 (m, 6H, $H^{17}$ + $H^{18}$ + $H^{19}$), 1.70 (m, 2H, $H^3$), 2.03, 2.10 (dt, 2H, J 7.51 and 7.32 Hz, $H^7$ + $H^{7'}$), 2.26-2.37 (m, 7H, $H^2$ + $H^4$ + $H^{13'}$ + $H^{16}$), 2.53 (m, 1H, $H^{13}$), 2.78 (dt, 1H, J 6.06 and 5.85 Hz, $H^{12}$), 3.21 (dd, 1H, J 9.06 and 5.85 Hz, $H^{11}$), 3.68 (s, 3H, $COOCH_3$), 4.14 (m, 1H, $H^8$), 5.37 (ddd, 1H, J 1.16, 9.08 and 15.36 Hz, $H^{10}$), 5.41-5.57 (m, 4H, olefinic H), 5.90 (dd, 1H, J 6.20 and 15.35 Hz, $H^9$). Mass-spectrum of the tBDMSi derivative (m/z, % of related intensity): 448 ([M - S]+, 0.22), 391 ([448 - t-Bu]$^+$, 2.5), 307 ([C8-C20 - S]$^+$, 100), 285 ([C1-C8]$^+$, 4.5), 175 ([307 - t-BDMSiOH]$^+$, 6.1) .

Example III

The ability of hepoxilin analogs to inhibit agonist-evoked changes in the free intracellular calcium concentration of human neutrophils was assayed.

Human neutrophils were separated from fresh human blood by dextran sedimentation followed by centrifugation on either a Ficoll-Hypaque gradient (Boyum, J. Clin. Invest. 21:77-98, 1968) or a discontinuous plasma-Percoll gradient (Downey et al., J. Appl. Physiol. 64:728-741, 1988). Residual red blood cells were removed by lysis with $NH_4Cl$ and centrifugation. Washed cells were maintained at room temperature in RPMI 1640 (Sigma Chemical Co., St. Louis, MO) without $HCO_3^-$ (buffered to pH 7.3 using 10 mM-Na Hepes) at $10^7$ cells/ml until assayed. For assaying, cells [(1-2) X $10^7$ cells/ml] were suspended in medium containing NaCl (140 nM), KCl (5 nM), $MgCl_2$ (1 nM), $CaCl_2$ (1 nM), Hepes (10 nM) and glucose (10 nM), pH 7.3 The osmolarity of the media was adjusted to 290 ± 5 mosm.

Intracellular free calcium was measured using the fluorescent indicator Indo-1-AM (Molecular Probes, Inc., Eugene, OR). One ml of the freshly prepared neutrophil suspension (10 million cells) was loaded with 3 μl of a 1 mM solution

of Indo-1-AM in DMSO (final concentration of the dye being 3 μM) for a period of 30 minutes at 37°C. Tubes were inverted every five minutes. The acetoxymethyl ester (AM) group on the dye allowed its penetration into the cell where esterases cleave it (by saponification) to release the free acid form which is trapped inside the cell. Unloaded dye was removed by centrifugation, and the cells were resuspended in fresh RPMI 1640 (Sigma Chemical Co.). Dye-loaded cells were kept at room temperature on a rotator. For each measurement, 2 x $10^6$ cells were added to 1 ml of a clear medium (composition in mM: NaCl 140, KCl 5, $MgCl_2$ 1, $CaCl_2$ 1, Hepes sodium free 10 and glucose 10, pH 7.3, osmolarity 290± 5) in a temperature-controlled plastic cuvette (Diamed Lab., Canada) at 37°C. The cell suspension was continuously stirred. Fluorescence was continuously monitored with a Perkin-Elmer fluorescence spectrophotometer , model 650-40 (Perkin-Elmer, Norwalk, CT) and recorded on an LKB model 2210 chart recorder (Pharmacia, Sweden) set at 2 cm/min. The excitation wavelength was set at 331 nm, the emission wavelength set at 410 nm, and slits of excitation and emission were set at 3 and 15 respectively. Temperature of the cuvette was maintained at 37°C ±2°C with water (at 37°C) circulating underneath the cuvette. An equilibrium period of approximately 2 to 5 minutes was allowed before addition of various agents to have a flat baseline signal. Intracellular free calcium was calculated using the following formula:

$$[Ca^{2+}]i = 250\text{ nM x }\frac{(F-F_{min})}{(F_{max}-F)}$$

All fluorescence values were measured relative to an $MnCl_2$-quenched signal determined as follows: 250 nM is the $K_d$ of Indo-1-AM, F is the relative fluorescence measurement of sample. $F_{max}$ was determined by exposing cells to the calcium ionophore ionomycin (Sigma Chemical Co.) at $10^{-6}$ M final concentration. After $F_{max}$ was determined, $MnCl_2$ at 3 mM final concentration was added to totally quench the Indo-1-AM signal and $F_{Mn}$ was obtained. $F_{min}$ was obtained as follows: 1/12 x ($F_{max}$ - $F_{Mn}$) + $F_{Mn}$. Calibration was performed on each sample. Hepoxilin analogs $HxA_3$ and other agonists (fMLP, PAF, $LTB_4$ and thapsigargin) were added to the cuvette as a 1000x concentrate in DMSO. Hepoxilin analogs HA921-924 were tested first at three concentrations (0.05, 0.1 and 0.5 μg/ml) in DMSO or the DMSO vehicle alone, followed five minutes later by each of the agonists at one concentration, i.e. $HxA_3$, 3 μg/ml; FMLP, 1 X $10^{-9}$M; $LTB_4$, 2 ng/ml; PAF, 1 ng/ml and thapsigargin, 100 ng/ml). Alteration of agonist response by the hepoxilin analogs was evaluated by comparison of response in the absence of analog (DMSO vehicle alone) with that observed in the presence of analog.

The results of the assays, shown in the Table, demonstrate that HA-921, HA-922 and HA-923 at the higher concentrations (1.4 μM) inhibit the stimulation of intracellular calcium induced by fMLP, $LTB_4$, hepoxilin $A_3$ and thapsigargin. The analog HA-924 is shown to inhibit hepoxilin $A_3$ and thapsigargin.

TABLE

| Hepoxilin analog (μg/ml) | Agonist* | free $[Ca^{2+}]_i$ (nM) | | | |
|---|---|---|---|---|---|
| | | **HA-921** | **HA-922** | **HA-923** | **HA-924** |
| 0 | fMLP | 580 | 871 | 741 | |
| 0.05 | " | 260 | 820 | 129 | |
| 0.1 | " | 420 | 461 | 516 | |
| 0.5 | " | 280 | 435 | 161 | |
| 0 | PAF | 800 | 1307 | 645 | |
| 0.05 | " | 980 | 1512 | 806 | |
| 0.1 | " | 840 | 2000 | 806 | |
| 0.5 | " | 440 | 615 | 1516 | |
| 0 | $LTB_4$ | 520 | nd | 290 | |
| 0.05 | " | 340 | 1076 | 548 | |
| 0.1 | " | 580 | nd | 64 | |
| 0.5 | " | 380 | 384 | nd | |
| 0 | $HxA_3$ | 300 | 1333 | 1451 | 638 |
| 0.05 | " | 240 | 333 | 193 | 381 |

*Agonist concentrations used were: FMLP, 1 × $10^{-9}$M; PAF, 1 ng/mL; $LTB_4$, 2 ng/mL; $HxA_3$, 3 μg/mL; Thapsigargin, 100 ng/mL.

TABLE (continued)

| Hepoxilin analog (μg/ml) | Agonist* | free $[Ca^{2+}]_i$ (nM) | | | |
|---|---|---|---|---|---|
| | | **HA-921** | **HA-922** | **HA-923** | **HA-924** |
| 0.1 | " | 140 | 256 | 193 | 273 |
| 0.5 | " | 320 | 128 | 322 | 26 |
| 0 | Thapsigargin | 1460 | 948 | 774 | 835 |
| 0.05 | " | 840 | 1256 | 2354 | 361 |
| 0.1 | " | 940 | 1051 | 612 | 250 |
| 0.5 | " | 280 | 358 | 225 | 39 |

*Agonist concentrations used were: FMLP, $1 \times 10^{-9}$M; PAF, 1 ng/mL; $LTB_4$, 2 ng/mL; $HxA_3$, 3 μg/mL; Thapsigargin, 100 ng/mL.

Statistical analyses of these experiments showed that analogs HA-923 and HA-924 have $IC_{50}$s of 0.52 μM and 0.30 μM, respectively, compared to $IC_{50}$s for the $HxA_3$ (8S) epimer of 8.6 μM and 1.5 μM for the $HxA_3$ (8R) epimer, indicating that the analogs are about 3-5 times more potent than the natural compound.

Example IV

The ability of hepoxilin analogs HA-923 and HA-924 to compete with hepoxilin $A_3$ for binding sites on neutrophils was assayed using $[^3H_6]$-hepoxilin $A_3$ (8S) methyl ester.

The hepoxilins were tritated using a modification of the procedures described in Vasiljeva et al. Tetrahedron 49: 4099, 1993 and Demin et al. Bioorg. Khim. 16:1125, 1990. The triacetylenic analog of $HxB_3$ (methyl 10(R/S)-hydroxy-11(S),12(S)-epoxy-5,8,14-eixosatriynoate) was hydrogenated and tritiated using Lindlar catalyst in 4:1 (w/w) instead of 1:1 in the presence of quinoline and 100% tritium gas (1; numbers refer to the scheme shown in Figure 3). The resulting complex mixture contained two epimeric and over-tritiated products of $[^3H_6]$-$HxB_3$ that were separated using argentation thin layer chromotography. The resulting $[^3H_6]$-$HxB_3$ epimers were separated from each other using straight phase HPLC. The ratio between the epimers was 65:35 with a preference for the (R) epimer as in the starting triacetylene mixture (2) The radiochemical purity of each epimer was >97% and the specific activity was approximately 169 Ci/mmol (2).

To obtain the corresponding epimer of $[^3H_6]$-$HxA_3$ (3) from $[^3H_6]$-$HxB_3$ (2) a reaction of $[^3H_6]$-$HxB_3$ under Mitsunobu conditions (diethylazodicarboxylate (DEAD)/triphenylphosphine/benzoic acid in THF at 20°C ) resulted in a mixture of $[^3H_6]$-$HxA_3$ and $[^3H_6]$-$HxB_3$ benzoates followed by treatment with MeONa in MeOH to hydrolyze the benzoates. For both epimers of $[^3H_6]$-$HxB_3$ (2) the $S_N2$ and $S_N2'$-type displacements of intermediate complexes (4) by benzoate anion gave an inverted epimer of $[^3H_6]$-$HxB_3$ and the two $[^3H_6]$-$HxA_3$ epimers (3) with preference for the epimer formed by attack of benzoate anion from the side opposite to the leaving groups as shown in Figure 3. Only (10R)-$[^3H_6]$-$HxB_3$ rearranges into $[^3H_6]$-$HxA_3$ epimeric mixture with good yield (up to 41%) affording a 6:1 mixture of (8R)-$[^3H_6]$-$HxA_3$ (3a) and (8S)-$[^3H_6]$-$HxA_3$ (3b) respectively; (10S-$[^3H_6]$-$HxB_3$ gives the (10R)-$[^3H_6]$-$HxB_3$ with only 7% of a 1:6 mixture of (8R)- and (8S)-$[^3H_6]$-$HxA_3$ (3a,b). Thus an actual 65:35 mixture of $[^3H_6]$-(10R) and (10S)-$HxB_3$ (2a,b) is rearranged into the mixture of $[^3H_6]$-(10R) and (10S)-$HxB_3$ (2a,b) and $[^3H_6]$-(8R) and (8S)-$HxA_3$ (3a,b) with a 3:1 ratio between the latter at an overall yield of 28%.

Yield of the (8S)-$[^3H_6]$-$HxA_3$ was increased by reacting the mixture once more under the Mitsunobu conditions to invert all (8R)-$[^3H_6]$-$HxA_3$ (3a) into (8S)-$[^3H_6]$-$HxA_3$ (3b), and to simultaneously obtain an additional quantity of (8R)-$[^3H_6]$-$HxA_3$ (3a) from (10R)-$[^3H_6]$-$HxB_3$ (2a). These conditions resulted in a mixture containing the two $[^3H_6]$-$HxA_3$ (3a, b) epimers in almost equal ratio. The epimers were separated on a straight-phase HPLC column resulting in pure individual [3H]-$HxA_3$ (3a and 3b) in reasonable yield and good radiochemical purity.

Neutrophil suspensions (prepared as described previously) containing 2 x $10^6$ cells in 1 ml total volume of assay medium (as described in Example III) were added to $[^3H_6]$-$HxA_3$(8S)-Me (32,000 cpm, specific activity 169 Ci/mmol) in the absence or presence of 0.5 or 1 μg of unlabelled hepoxilin to measure non specific binding. In separate tubes, 0.5 μg of cyclopropyl analogs HA-923 or HA-924 was added to the tritiated hepoxilin. The tubes were incubated at 37°C for 1 hour. After incubation the reaction mixture was rapidly filtered through Whatman GF/B glass fiber filters (Whatman Biosystems, Maidstone, England) that had been prewashed with the assay medium. The tubes were rinsed three times with ice-cold assay medium. The radioactivity retained on the filters was counted in 10 ml of ECOLITE scintillation fluid (ICN, St. Laurent, Quebec) in a Beckman (Model LS 3800) scintillation spectrophotometer (Beckman, Irvine, CA).

Figure 4 illustrates that specific binding of hepoxilin $A_3$ occurs in human neutrophils and that hepoxilin analogs

HA-923 and HA-924 inhibit the binding of binding of tritiated hepoxilin $A_3$.

Although the foregoing invention has been described in some detail by way of illustration and example for purposes of clarity of understanding, it will be evident that certain changes and modifications may be practiced within the scope of the appended claims. For example, the synthetic methodology may be modified by the ordinarily skilled practioner to produce other hepoxilin analogs, the activity of which can be readily assayed.

## Claims

**1.** A compound selected from:

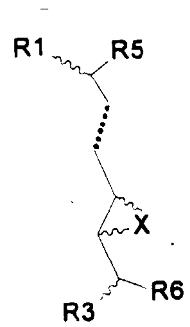

;

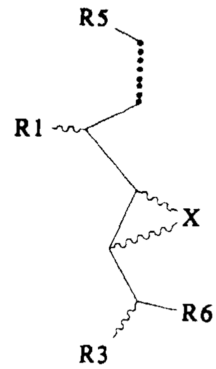

;

and pharmaceutically acceptable salts thereof, wherein:

X is $CH_2$, NH, or S;
R1 is OH, $CH_3$, $CH_2OH$, $N_3$ OR $CH_2N_3$;
R3 is H or $CH_3$;
R5 is Y-R2, wherein Y is a six-carbon chain optionally containing up to three double or triple bonds or a mixture of double and triple bonds up to a maximum of three;
R2 is $C_1$-$C_{10}$ alkyl-OH, $C_1$-$C_{10}$ alkyl-$N_3$ or COOR4;
R4 is H, $C_1$-$C_{10}$ alkyl, $C_5$-$C_6$ cycloalkyl, or $C_5$-$C_6$ aryl;
R6 is a seven-carbon chain optionally containing up to three double or triple bonds or a mixture of double and triple bonds up to a maximum of three; and
......... is a single, double or triple bond.

**2.** A compound according to claim 1 wherein R2 is alkyl-$N_3$, alkyl-OH or COOR4, wherein R4 is $C_5$-$C_{10}$ alkyl or $C_5$-$C_6$ aryl.

**3.** A compound according to claim 1 selected from:

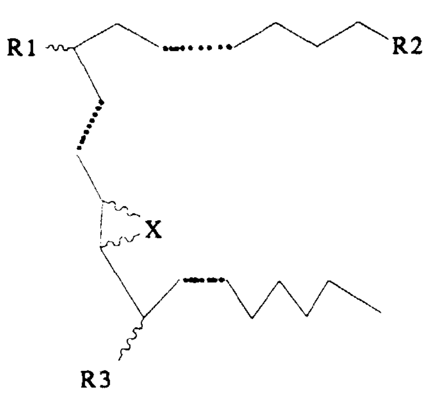

or

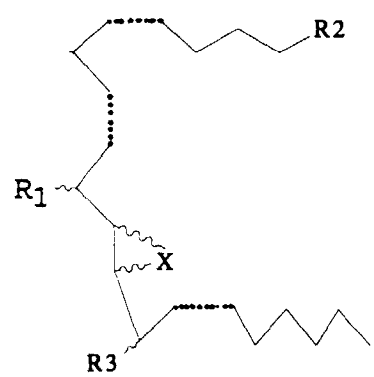

and pharmaceutically acceptable salts thereof, wherein X, R1, R2, R3, R4 and ...... are as defined in claim 1.

4. A compound according to claim 3 wherein X is $CH_2$.

5. A compound according to claim 3 wherein R1 is OH.

6. A compound according to claim 3 wherein R3 is H.

7. A compound according to claim 3 wherein R2 is COOH or $COOCH_3$.

8. A compound according to claim 3 wherein said compound is

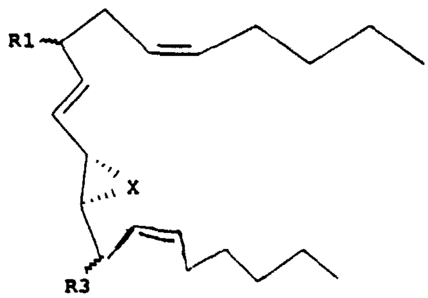

,

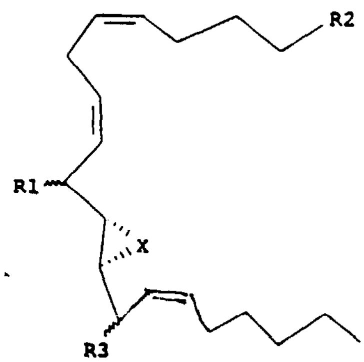

or a pharmaceutically acceptable salt thereof.

9. A compound according to claim 8 wherein said compound is any of:

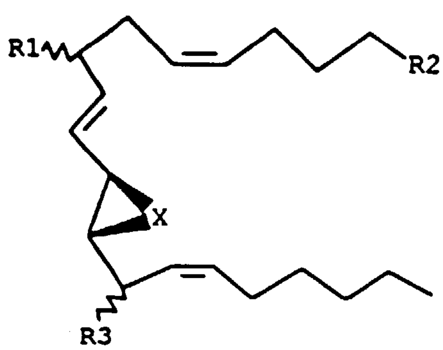

,

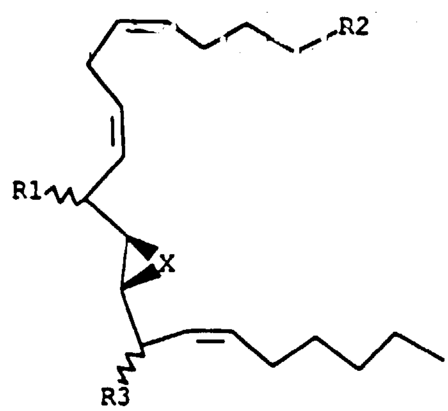

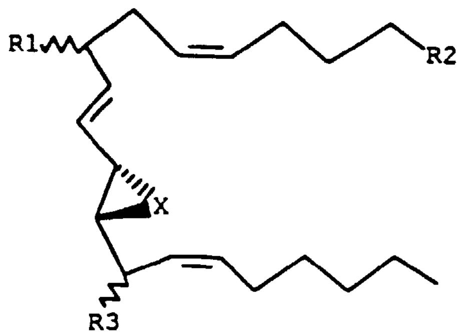

,

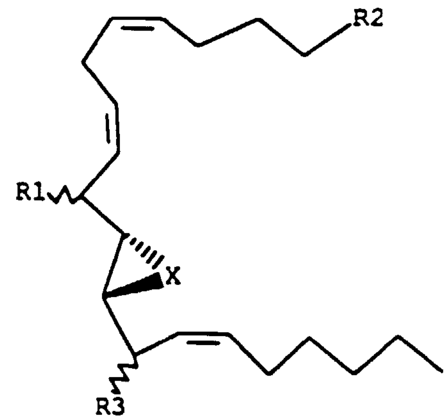

,

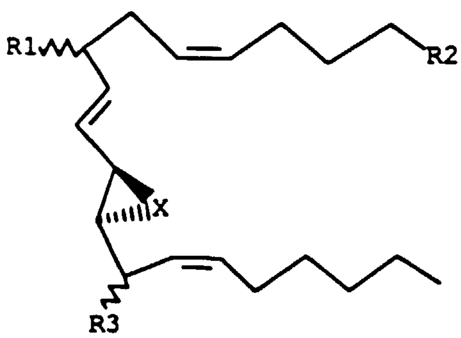

,

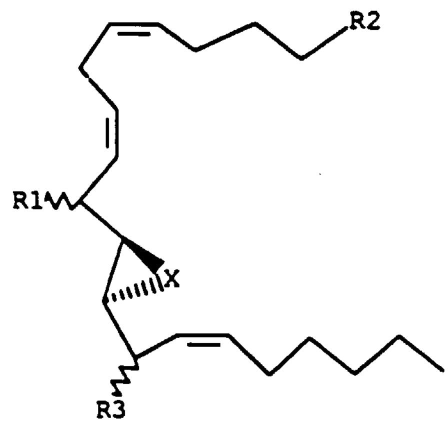

,

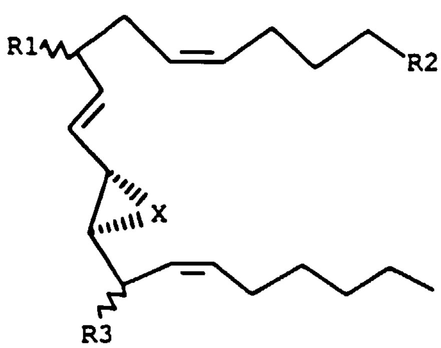

,

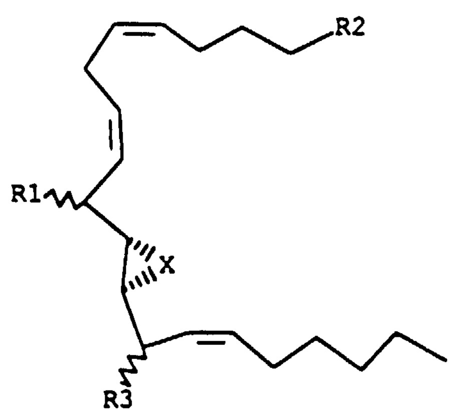

or a pharmaceutically acceptable salt thereof.

**10.** A compound according to claim 9 wherein R1 is OH; R2 is COOH or $COOCH_3$ and R3 is H.

**11.** A compound according to claim 3 wherein R4 is $C_5$-$C_{10}$ alkyl, $C_5$-$C_6$ cycloalkyl or $C_5$-$C_6$ aryl.

**12.** A compound according to claim 11 wherein ......... is a double bond.

**13.** A compound according to claim 3 wherein R2 is alkyl-$N_3$, or alkyl-OH.

**14.** A compound according to claim 3 selected from:

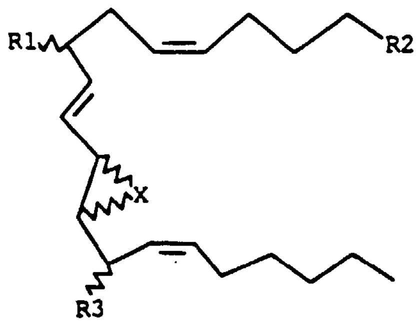

,

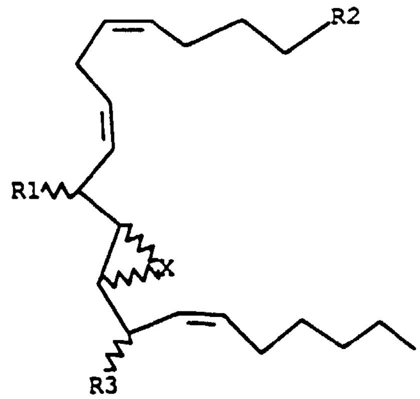

and pharmaceutically acceptable salts thereof, wherein:

X R1 and R3 are as defined in claim 1;

R2 is COOR4;

R4 is H or lower alkyl.

**15.** A compound according to claim 14 wherein X is $CH_2$.

**16.** A compound according to claim 15 wherein R1 is OH, R2 is COOH or $COOCH_3$, and R3 is H.

**17.** A compound according to claim 14 wherein R1 is OH, R2 is COOH or $COOCH_3$, and R3 is H.

**18.** A compound according to claim 14 selected from:

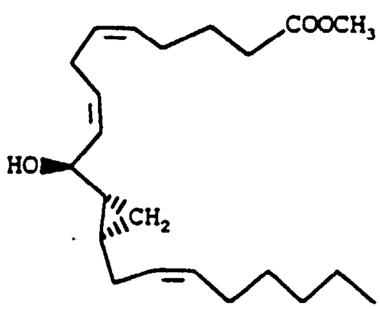

,

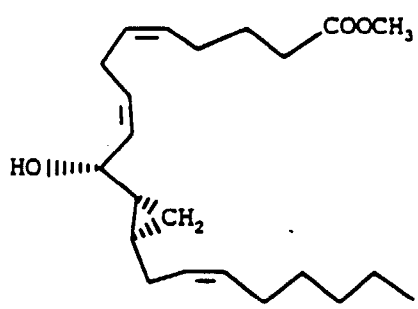

,

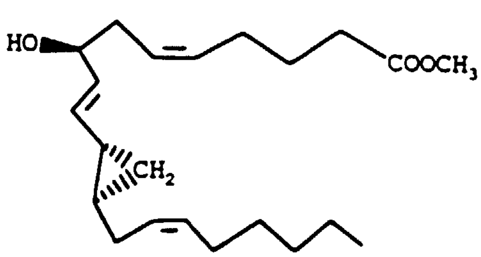

,

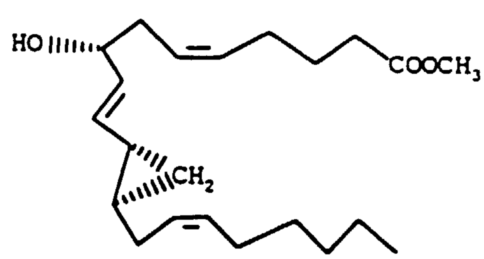

and pharmaceutically acceptable salts thereof.

**19.** A pharmaceutical composition comprising a compound according to claim 1 in combination with a pharmaceutically acceptable vehicle.

**Patentansprüche**

**1.** Verbindung ausgewählt aus:

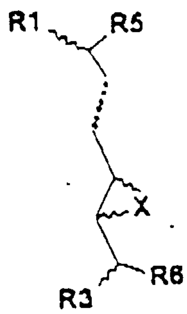

;

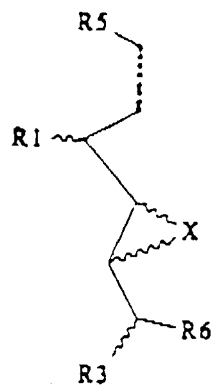

;

und pharmazeutisch annehmbaren Salzen davon, wobei:

X $CH_2$, NH oder S ist;
R1 OH, $CH_3$, $CH_2OH$, $N_3$ oder $CH_2N_3$ ist;
R3 H oder $CH_3$ ist;
R5 Y-R2 ist, wobei Y eine Kette mit sechs Kohlenstoffatomen ist, die gewünschtenfalls bis zu drei Doppel- oder Dreifach-Bindungen oder eine Mischung aus Doppel- und Dreifach-Bindungen bis zu einem Maximum von drei enthält;
R2 $C_1$-$C_{10}$-Alkyl-OH, $C_1$-$C_{10}$-Alkyl-$N_3$ oder COOR4 ist;
R4 H, $C_1$-$C_{10}$-Alkyl, $C_5$-$C_6$-Cycloalkyl oder $C_5$-$C_6$-Alryl ist;
R6 eine Kette mit sieben Kohlenstoffatomen ist, die gewünschtenfalls bis zu drei Doppel- oder Dreifach-Bindungen oder eine Mischung von Doppel- und Dreifach-Bindungen bis zu einem Maximum von drei enthält; und
........... eine Einfach-, Doppel- oder Dreifach-Bindung ist.

**2.** Verbindung nach Anspruch 1, bei der R2 Alkyl-$N_3$, Alkyl-OH oder COOR4 ist, wobei R4 $C_5$-$C_{10}$-Alkyl oder $C_5$-$C_6$-Aryl ist.

**3.** Verbindung nach Anspruch 1, ausgewählt aus:

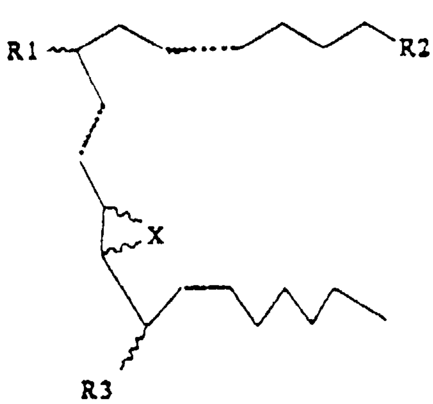

oder

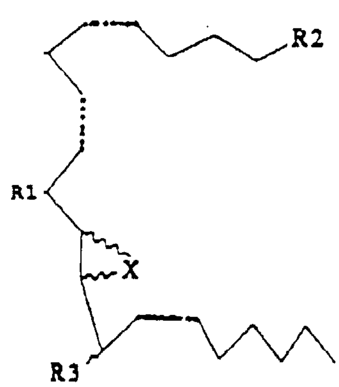

und pharmazeutisch annehmbaren Salzen davon, wobei X, R1, R2, R3, R4 und wie in Anspruch 1 definiert sind.

**4.** Verbindung nach Anspruch 3, bei der X $CH_2$ ist.

**5.** Verbindung nach Anspruch 3, bei der R1 OH ist.

**6.** Verbindung nach Anspruch 3, bei der R3 H ist.

**7.** Verbindung nach Anspruch 3, bei der R2 COOH oder $COOCH_3$ ist.

**8.** Verbindung nach Anspruch 3, bei der die Verbindung

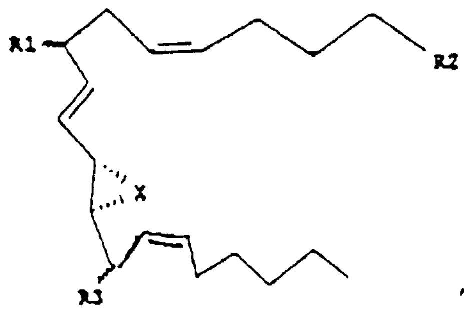

,

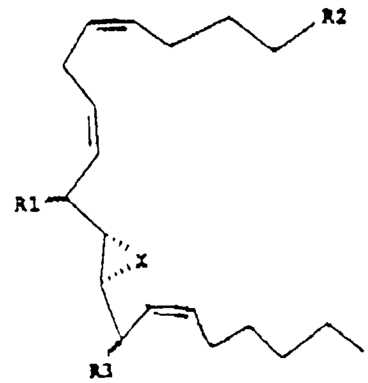

oder ein pharmazeutisch annehmbares Salz davon ist.

**9.** Verbindung nach Anspruch 8, bei der die Verbindung irgendeine der folgenden:

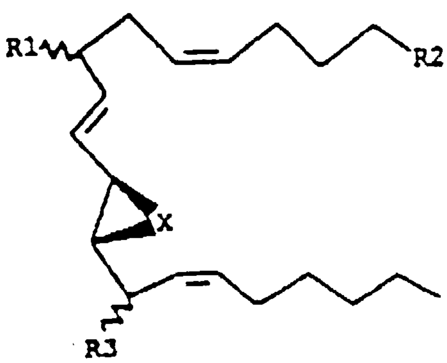

,

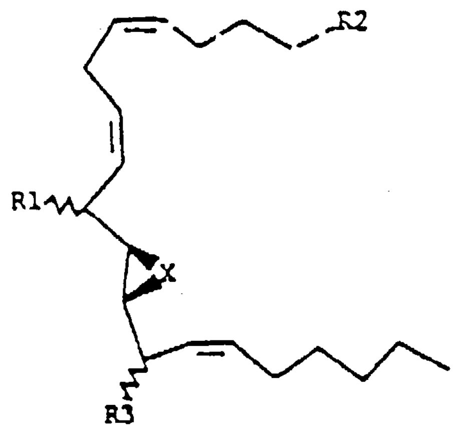

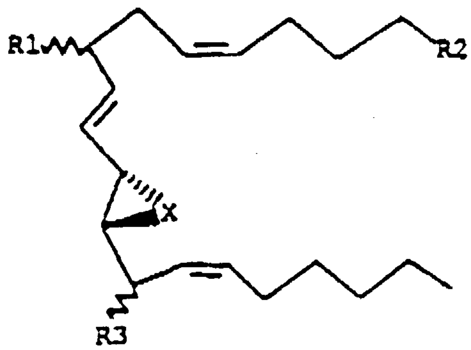
,
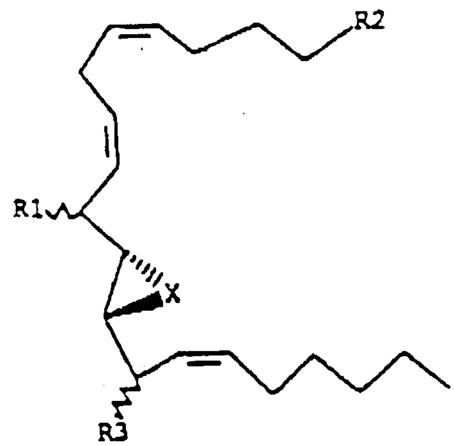
,
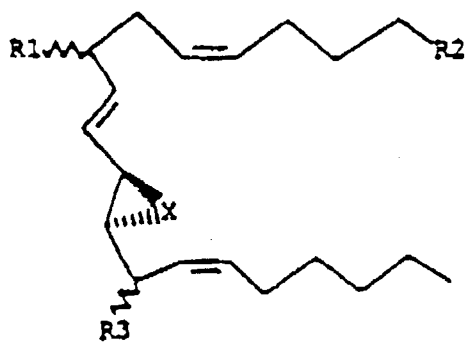
,
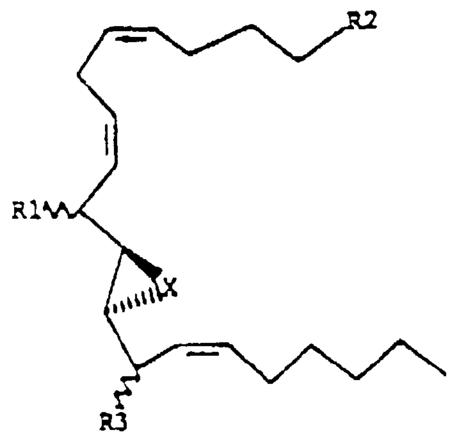
,
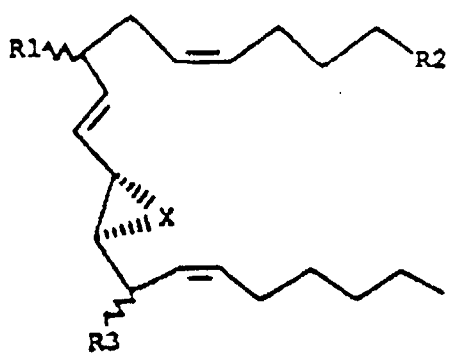
,
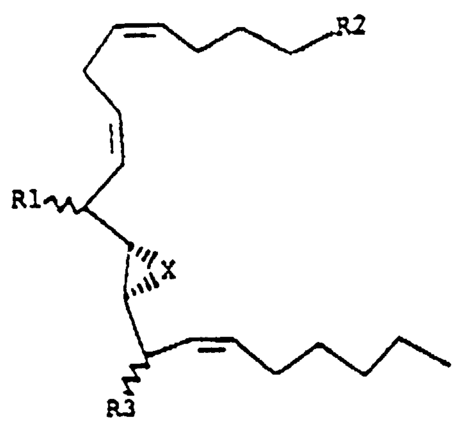

oder ein pharmazeutisch annehmbares Salz davon ist.

**10.** Verbindung nach Anspruch 9, bei der R1 OH ist; R2 COOH oder $COOCH_3$ ist und R3 H ist.

**11.** Verbindung nach Anspruch 3, bei der R4 $C_5$-$C_{10}$-Alkyl, $C_5$-$C_6$-Cycloalkyl oder $C_5$-$C_6$-Aryl ist.

**12.** Verbindung nach Anspruch 11, bei der eine Doppelbindung ist.

**13.** Verbindung nach Anspruch 3, bei der R2 Alkyl-$N_3$ oder Alkyl-OH ist.

**14.** Verbindung nach Anspruch 3, ausgewählt aus:

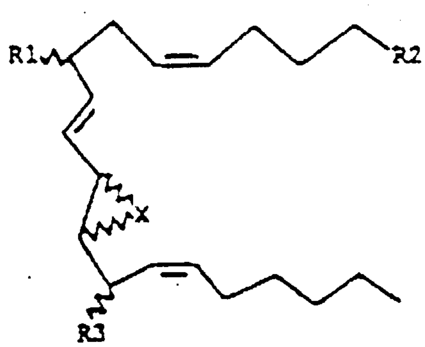

,

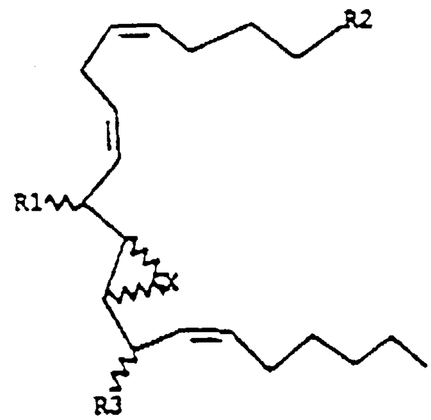

und pharmazeutisch annehmbaren Salzen davon, wobei:

X, R1 und R3 wie in Anspruch 1 definiert sind;
R2 COOR4 ist;
R4 H oder ein niederes Alkyl ist.

**15.** Verbindung nach Anspruch 14, bei der X $CH_2$ ist.

**16.** Verbindung nach Anspruch 15, bei der R1 OH ist, R2 COOH oder $COOCH_3$ ist und R3 H ist.

**17.** Verbindung nach Anspruch 14, bei der R1 OH ist, R2 COOH oder $COOCH_3$ ist und R3 H ist.

**18.** Verbindung nach Anspruch 14, ausgewählt aus:

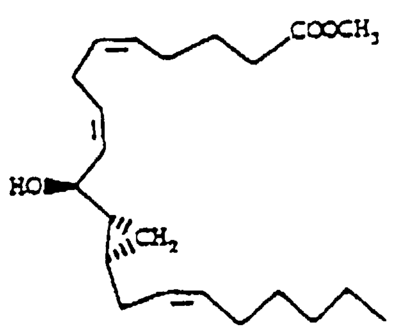

,

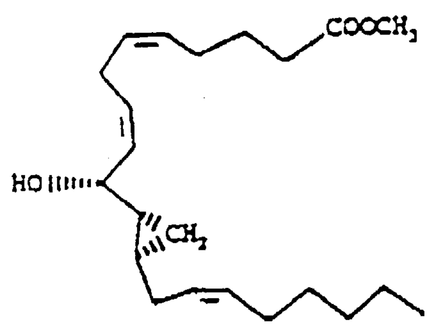

,

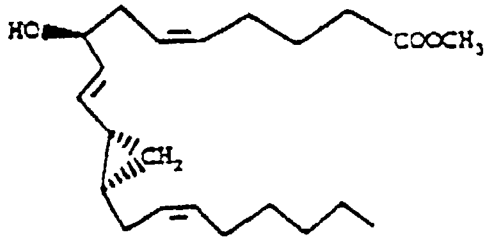

,

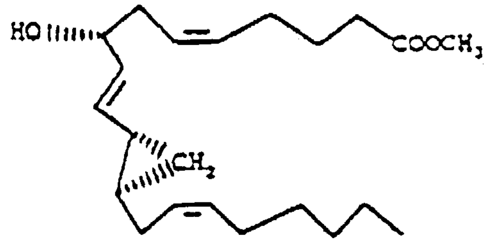

und pharmezeutisch annehmbaren Salzen davon.

**19.** Pharmazeutische Zusammensetzung aufweisend eine Verbindung nach Anspruch 1 in Kombination mit einem pharmazeutisch annehmbaren Träger.

## Revendications

**1.** Composé choisi parmi

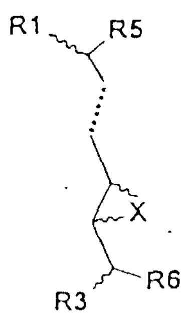

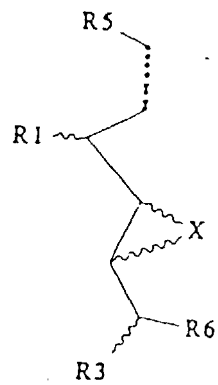

et ses sels acceptables d'un point de vue pharmaceutique, où

X est $CH_2$, NH ou S ;
R1 est OH, $CH_3$, $CH_2OH$, $N_3$ ou $CH_2N_3$ ;
R3 est H ou $CH_3$ ;
R5 est Y-R2, où Y est une chaîne à six atomes de carbone, contenant éventuellement jusqu'à trois doubles ou triples liaisons, ou un mélange de doubles et de triples liaisons, jusqu'à un maximum de trois ;
R2 est un radical (alkyle en $C_1$-$C_{10}$)-OH, (alkyle en $C_1$-$C_{10}$)-$N_3$ ou COOR4 ;
R4 est H ou un radical alkyle en $C_1$-$C_{10}$, cycloalkyle en $C_5$-$C_6$, ou aryle en $C_5$-$C_6$ ;
R6 est une chaîne à sept atomes de carbone contenant éventuelleument jusqu'à trois doubles ou triples liaisons ou un mélange de doubles et de triples liaisons jusqu'à un maximum de trois ; et
.......... est une liaison simple, double ou triple.

**2.** Composé selon la revendication 1, dans lequel R2 est un radical alkyl-$N_3$, alkyl-OH ou COOR4, où R4 est un radical alkyle en $C_5$-$C_{10}$ ou aryle en $C_5$-$C_6$.

**3.** Composé selon la revendication 1, choisi parmi

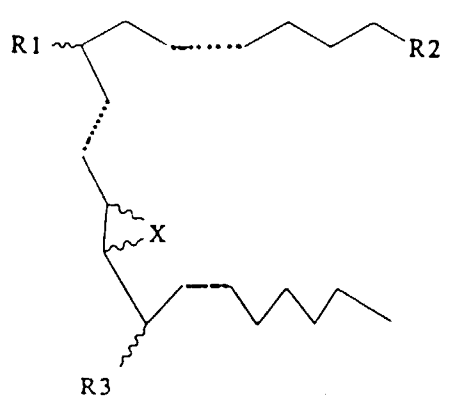

ou

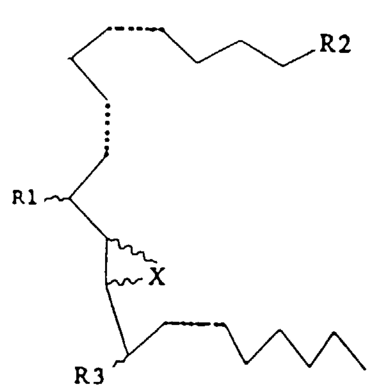

et ses sels acceptables d'un point de vue pharmaceutique, où X, R1, R2, R3, R4 et ......sont tels que définis dans la revendication 1.

**4.** Composé selon la revendication 3, dans lequel X est $CH_2$.

**5.** Composé selon la revendication 3, dans lequel R1 est OH.

**6.** Composé selon la revendication 3, dans lequel R3 est H.

**7.** Composé selon la revendication 3, dans lequel R2 est COOH ou $COOCH_3$.

**8.** Composé selon la revendication 3, dans lequel ledit composé est

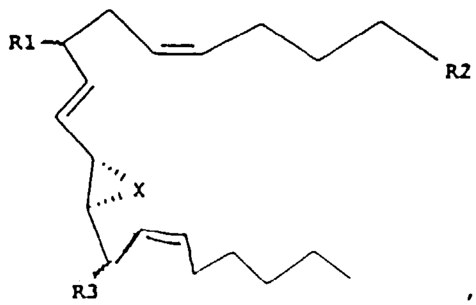

,

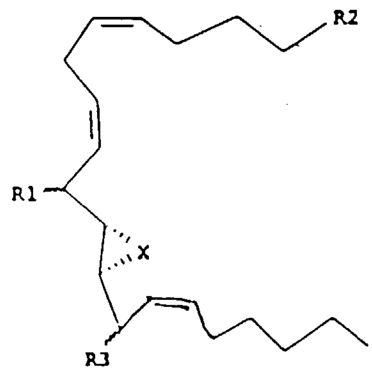

ou l'un de ses sels acceptables d'un point de vue pharmaceutique.

**9.** Composé selon la revendication 8, dans lequel ledit composé est l'un quelconque des composés suivants :

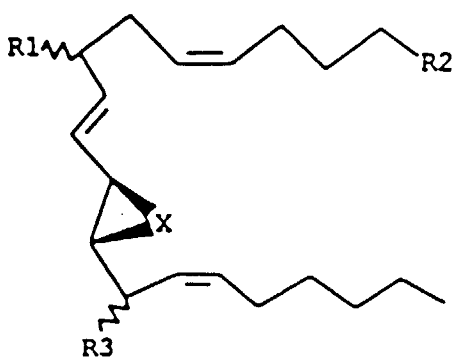

,

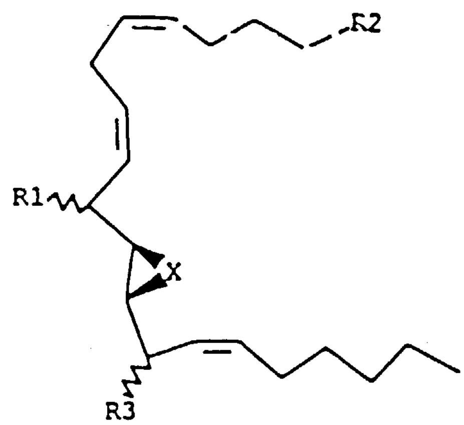

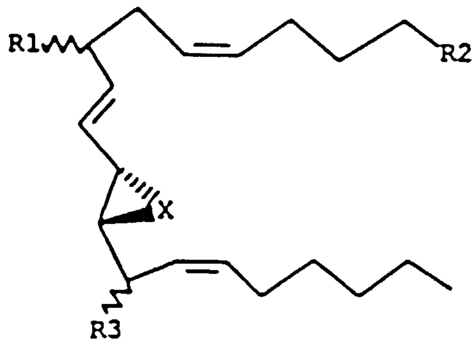
,
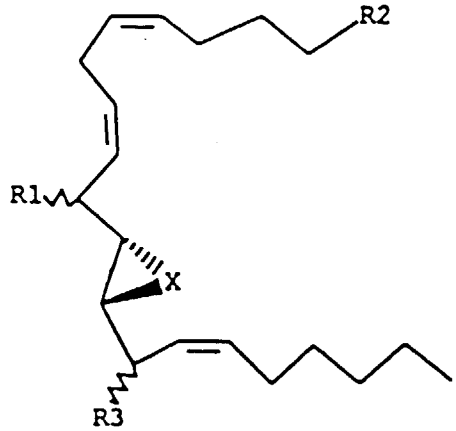
,

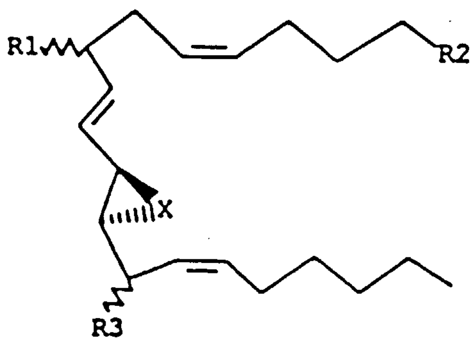
,
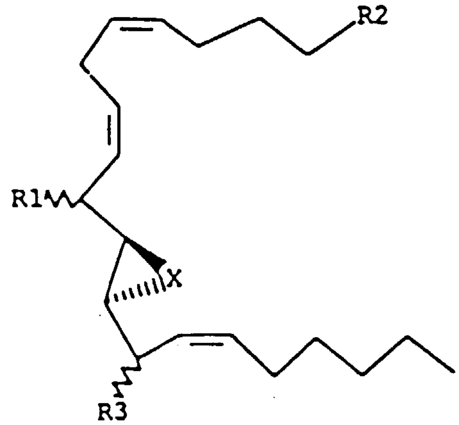
,

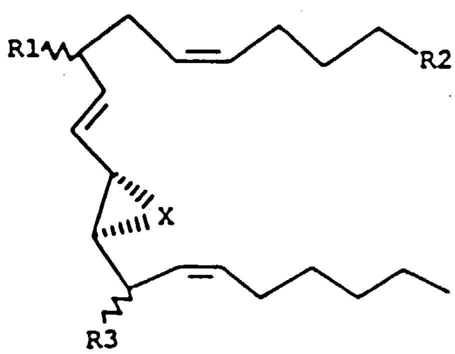
,
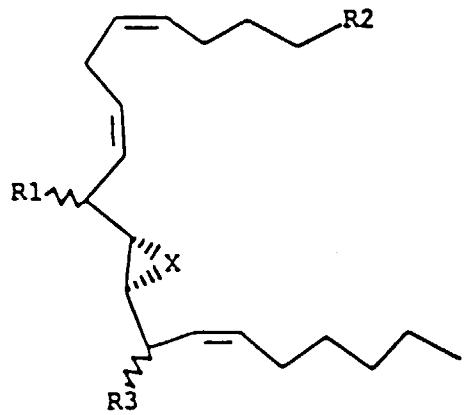

ou l'un de ses sels acceptables d'un point de vue pharmaceutique.

**10.** Composé selon la revendication 9, dans lequel R1 est OH, R2 est COOH ou $COOCH_3$, et R3 est H.

**11.** Composé selon la revendication 3, dans lequel R4 est un radical alkyle en $C_5$-$C_{10}$, cycloalkyle en $C_5$-$C_6$ ou aryle en $C_5$-$C_6$.

**12.** Composé selon la revendication 11, dans lequel ...... est une double liaison.

**13.** Composé selon la revendication 3, dans lequel R2 est un radical alkyl-$N_3$ ou alkyl-OH.

**14.** Composé selon la revendication 3, choisi parmi

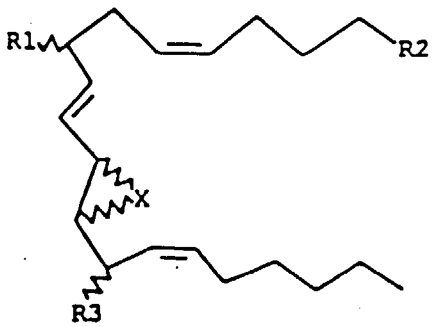

,

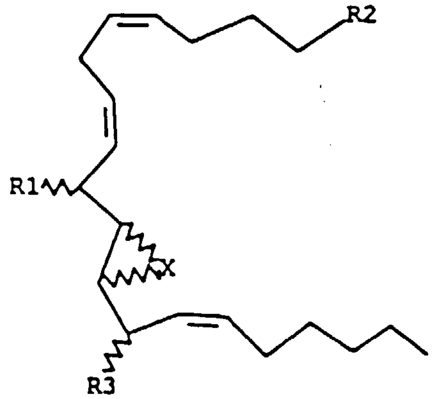

et ses sels acceptables d'un point de vue pharmaceutique, où

X, R1 et R3 sont tels que définis dans la revendication 1 ;
R2 est COOR4 ;
R4 est H ou un radical alkyle inférieur.

**15.** Composé selon la revendication 14, dans lequel X est $CH_2$.

**16.** Composé selon la revendication 15, dans lequel R1 est OH, R2 est COOH ou $COOCH_3$, et R3 est H.

**17.** Composé selon la revendication 14, dans lequel R1 est OH, R2 est COOH ou $COOCH_3$, et R3 est H.

**18.** Composé selon la revendication 14, choisi parmi

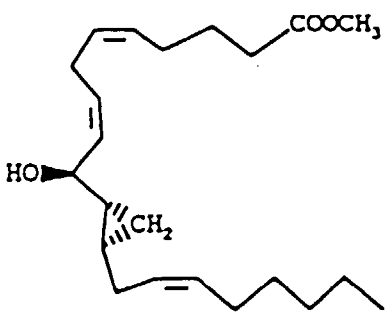

,

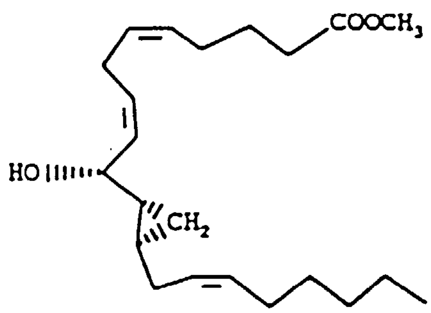

,

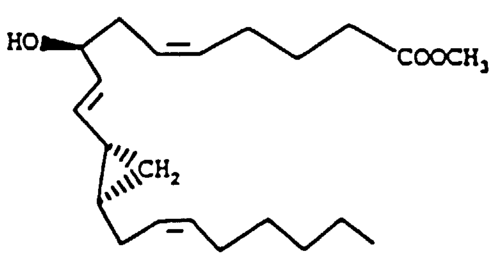

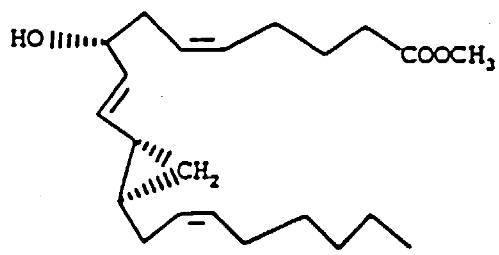

,

et ses sels acceptables d'un point de vue pharmaceutique.

19. Composition pharmaceutique comprenant un composé selon la revendication 1, en combinaison avec un véhicule acceptable d'un point de vue pharmaceutique.

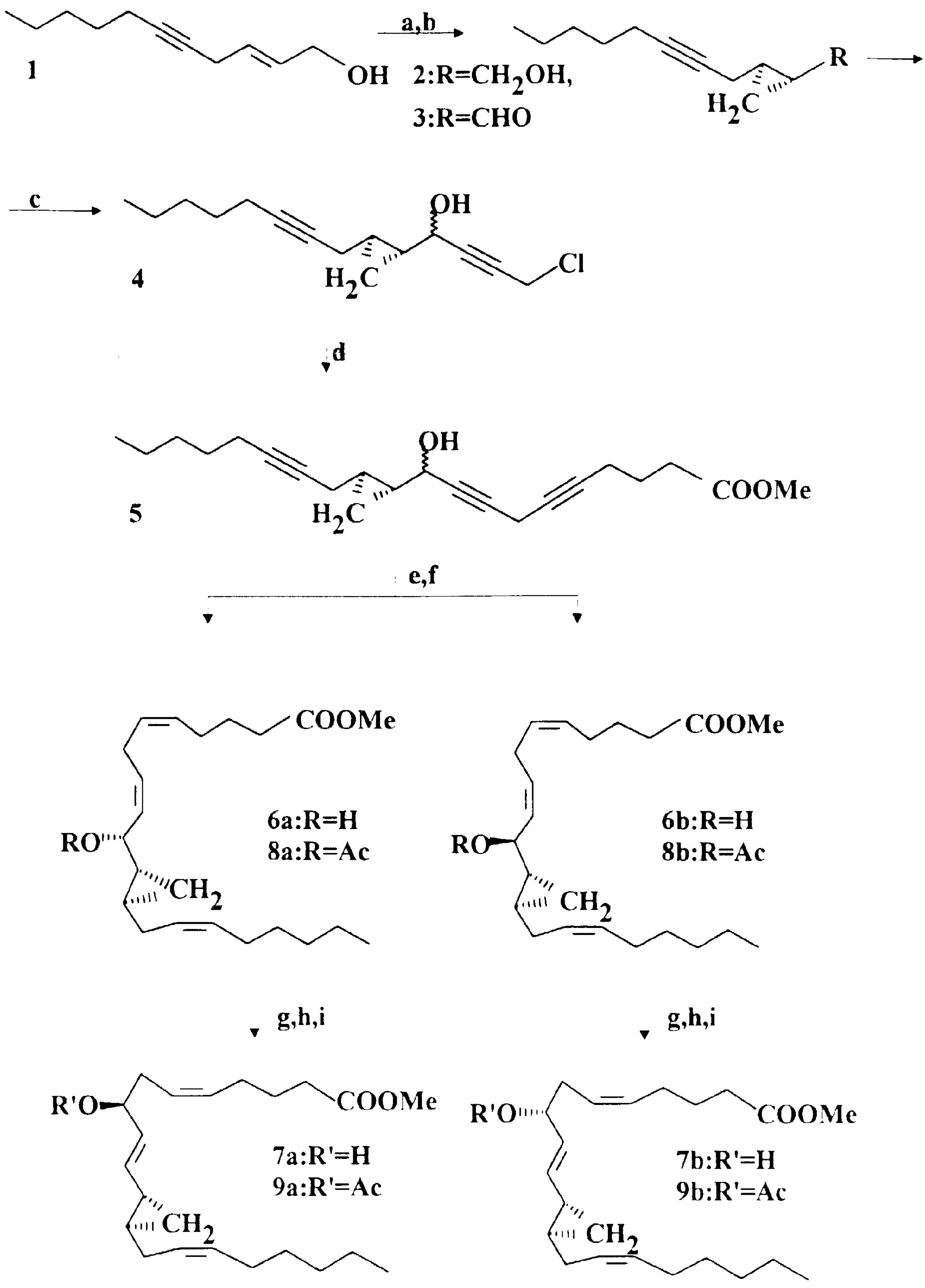
1
a,b
2:R=CH2OH,
3:R=CHO
c
4
d
5
e,f
6a:R=H
8a:R=Ac
6b:R=H
8b:R=Ac
g,h,i
g,h,i
7a:R'=H
9a:R'=Ac
7b:R'=H
9b:R'=Ac

*FIGURE 1*

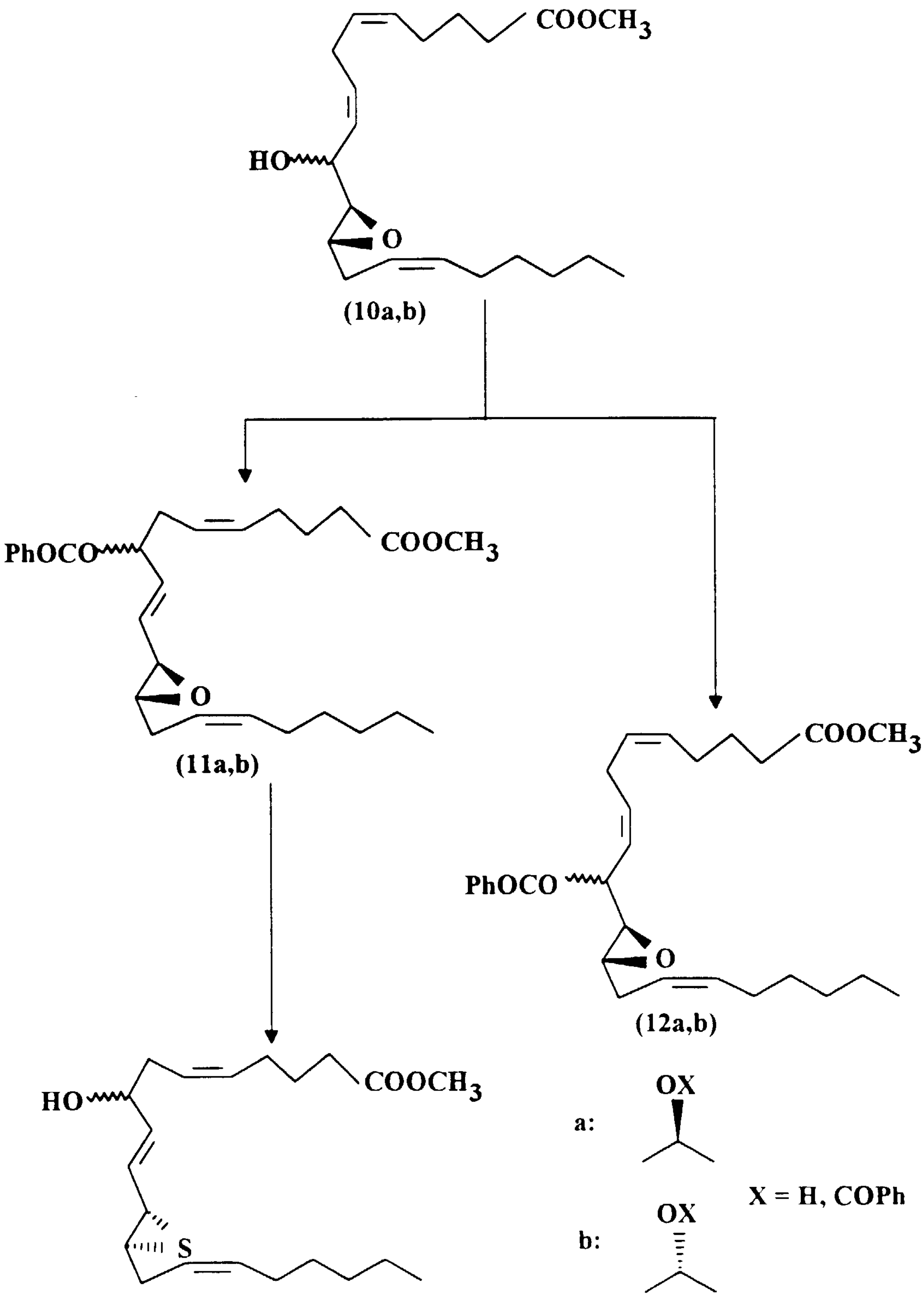

(10a,b)

(11a,b)

(12a,b)

(13a,b)

a:

b:

X = H, COPh

*FIGURE 2*

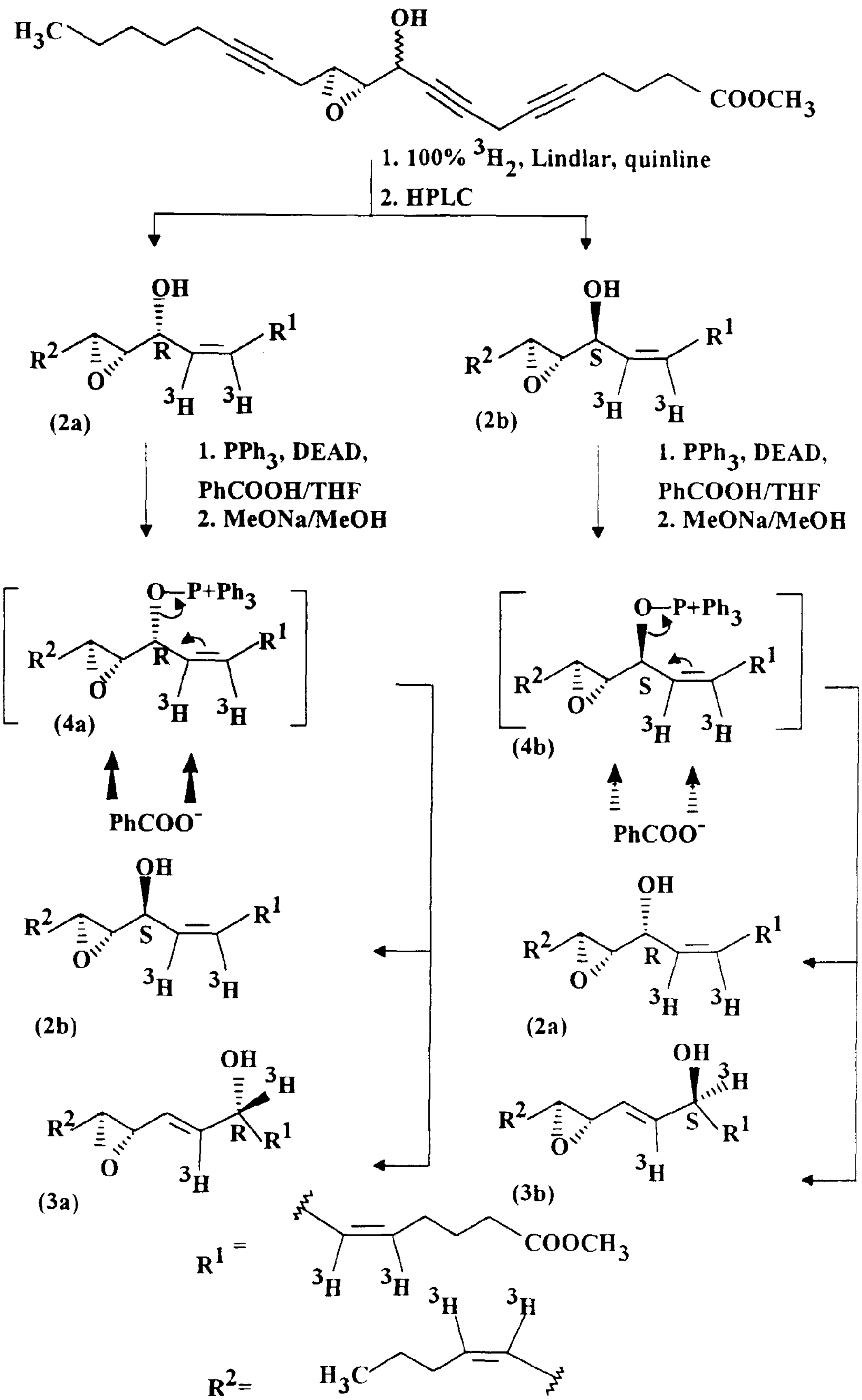
1. 100% ³H₂, Lindlar, quinline
2. HPLC
(2a)
(2b)
1. PPh₃, DEAD, PhCOOH/THF
2. MeONa/MeOH
1. PPh₃, DEAD, PhCOOH/THF
2. MeONa/MeOH
(4a)
(4b)
PhCOO⁻
PhCOO⁻
(2b)
(2a)
(3a)
(3b)

*FIGURE 3*

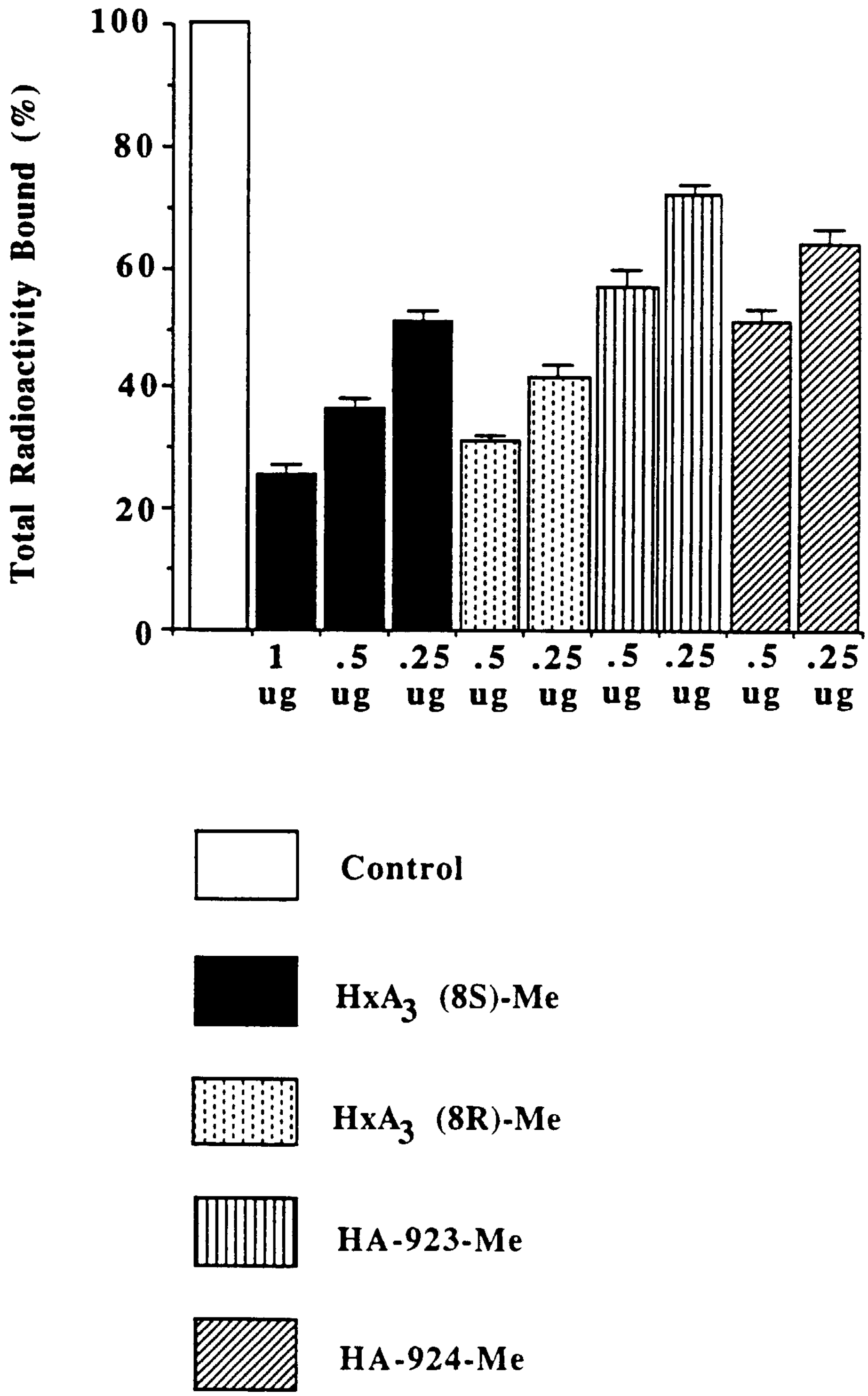
Total Radioactivity Bound (%)
100
80
60
40
20
0
1 ug
.5 ug
.25 ug
.5 ug
.25 ug
.5 ug
.25 ug
.5 ug
.25 ug
Control
HxA3 (8S)-Me
HxA3 (8R)-Me
HA-923-Me
HA-924-Me

# Figure 4